# EUROPEAN PATENT SPECIFICATION

(11) **EP 0 794 434 B1**
(45) Date of publication and mention of the grant of the patent: **06.04.2005**
(21) Application number: 96931261.0
(22) Date of filing: 20.09.1996
(51) Int. Cl.: G01N 33/53, G01N 33/538

(54) **METHOD FOR THE ANALYSIS OF ALLERGEN**
VERFAHREN ZUR ANALYSE VON ALLERGENEN
PROCEDE D'ANALYSE POUR ALLERGENE

(30) Priority: 22.09.1995 JP 26925795
(43) Date of publication of application: 10.09.1997
(73) Proprietor: ASAHI DENKA KOGYO KABUSHIKI KAISHA, Arakawa-ku Tokyo 116 (JP); MATSUNAGA, Tadashi, 2-40, Saiwai-cho, Fuchu-shi, Tokyo 183 (JP)
(72) Inventor: MATSUNAGA, Tadashi, Fuchu-shi, Tokyo 183 (JP)
(74) Representative: Minderop, Ralph H., Dr. rer. nat.
(86) International application number: PCT/JP1996/002707
(87) International publication number: WO 1997/011368

(56) References cited:
- EP-A- 0 498 124
- WO-A-92/19970
- JP-A- 7 043 364
- JP-B- 7 034 015

## Description

### TECHNICAL FIELD

The present invention relates to a method for assaying an allergen. According to the present invention, an allergen in a sample can be immunologically assayed by using a labeled antibody, a simple process of separating a complex of the allergen and the labeled antibody by an ion exchange chromatography, and measuring signals from the separated complex of the allergen and the labeled antibody.

### BACKGROUND ART

Allergies have now become a serious social problem, and the prevention or the treatment of such allergies is a matter of considerable concern. For example, allergen-reduced or allergen-free foods for patients suffering from food allergies have been developed as a means of preventing or treating allergies. Accordingly, an assay of allergens in foods or the like has become a very important technical subject.

Methods conventionally used to measure an allergen in food include, for example, a RAST (Radio-Allergo Sorbent Test) wherein an antibody labeled with a radio-active isotope is used and an allergen-antibody reaction is indirectly measured, an ELISA (Enzyme-Linked Immuno Sorbent Assay) wherein an antibody labeled with an enzyme is used and an allergen-antibody reaction is indirectly measured, and a PCA (Passive Cutaneous Anaphylaxis) wherein an allergy reaction of an *in vivo* level is detected on a skin of a rat. Nevertheless, in order to obtain the desired results, the procedures of the above methods are cumbersome and time-consuming (several hours to 1 day). Further, when a lot of samples are to be examined, it is necessary to divide the samples into groups and treat them batchwise, and therefore, such a procedure becomes even more cumbersome.

EP 0 357 869 A1 describes the reaction of an analyte, for example, an antigen, in a sample with a substance having affinity for the analyte, an antibody, for example, to obtain a reaction mixture. Than the free antibody is separated from the complex of the antigen and the antibody by HPLC on the basis of the different elution time.

As stated above, heretofore there was no appropriate method for rapidly and simply measuring allergens contained in foods, and therefore, the quality control necessary when preparing food for patients suffering from a food allergy was a problem. A method for electrochemically detecting allergen wherein an animal cell is used and a serotonin, which is a chemical mediator of an allergy reaction, is used as a marker is disclosed in Japanese Unexamined Patent Publication (Kokai) No. 6-288976.

Accordingly, the object of the present invention is to provide a simpler and more rapid method for quantitatively determining an allergen, in comparison with conventional methods for assaying an allergen.

Further, the object of the present invention is to provide a method which does not require a preparation of animal cells, in contrast to the above electrochemical- detecting method.

Other objects and advantages of the present invention will be apparent from the following description.

### DISCLOSURE OF INVENTION

The above object can be achieved by the present invention relating to a method for assaying an allergen, comprising the steps of:
(1) bringing a sample possibly containing an allergen to be examined into contact with a labeled antibody specific to the allergen to be examined under a condition wherein an antigen-antibody reaction between the allergen to be examined and the labeled antibody can be carried out;
(2) removing the labeled antibody not bound to the allergen to be examined, from a reaction mixture by an ion exchange chromatography, to obtain an eluted complex of the allergen to be examined and the labeled antibody from the ion exchange chromatography; said ion exchange chromatography being carried out under a condition that the complex of the allergen to be examined and the labeled antibody is not adsorbed on an ion exchanger in a range where the unreacted labeled antibody is adsorbed on the ion exchanger; and
(3) measuring a signal from an eluted complex of the allergen to be examined and the labeled antibody.

### BRIF DESCRIPTION OF DRAWINGS

Figure 1 shows a structural formula of a fluorescein isthiocyanate (FITC).
Figure 2 shows a reaction between an FITC and an IgE antibody.
Figure 3 is a graph illustrating changes of amounts of (DNP-BSA)-FITC labeled IgE antibody complexes with time.
Figure 4 is a chart illustrating a separation of (DNP-BSA)-FITC labeled IgE antibody complexes from unreacted FITC labeled IgE antibodies by a cation exchange chromatography.
Figure 5 is a chart illustrating a separation of (DNP-BSA)-FITC labeled IgE antibody complexes from unreacted labeled IgE antibodies by a cation exchange chromatography.
Figure 6 is a graph illustrating a reaction specificity of an FITC labeled anti-DNP-IgE antibody.
Figure 7 is a standard curve exhibiting a quantitative change of fluorescent strength derived from a (DNP-BSA)-FITC labeled IgE antibody complex.
Figure 8 is a chart illustrating a separation of wheat allergen-alkaline phosphatase labeled IgE antibody complexes from unreacted alkaline phosphatase labeled IgE antibodies by a cation exchange chromatography.
Figure 9 is a standard curve exhibiting a quantitative change of fluorescent strength derived from a wheat allergen-alkaline phosphatase labeled IgE antibody complex.
Figure 10 is a chart illustrating a separation of (β-lactoglobulin)-alkaline phosphatase labeled IgG antibody complexes from unreacted alkaline phosphatase labeled IgG antibodies by a cation exchange chromatography.
Figure 11 is a chart illustrating a separation of egg allergen-alkaline phosphatase labeled IgG antibody complexes from unreacted alkaline phosphatase labeled IgG antibodies by a cation exchange chromatography.
Figure 12 is a chart illustrating a separation of rice allergen-alkaline phosphatase labeled IgG antibody complexes from unreacted alkaline phosphatase labeled IgG antibodies by a cation exchange chromatography.
Figure 13 is a chart illustrating a separation of wheat allergen-alkaline phosphatase labeled IgG antibody complexes from unreacted alkaline phosphatase labeled IgG antibodies by a cation exchange chromatography.
Figure 14 is a chart illustrating a separation of buckwheat (soba) allergen-alkaline phosphatase labeled IgG antibody complexes from unreacted alkaline phosphatase labeled IgG antibodies by a cation exchange chromatography.
Figure 15 is a chart illustrating a separation of soybean allergen-alkaline phosphatase labeled IgG antibody complexes from unreacted alkaline phosphatase labeled IgG antibodies by a cation exchange chromatography.
Figure 16 is a standard curve exhibiting a quantitative change of fluorescent strength derived from a (β-lactoglobulin)-alkaline phosphatase labeled IgE antibody complex.
Figure 17 is a chart illustrating a separation of (β-lactoglobulin)-FITC labeled IgE antibody complexes from unreacted alkaline phosphatase labeled IgE antibodies by a cation exchange chromatography.
Figure 18 is a chart illustrating a separation of egg allergen-FITC labeled IgE antibody complexes from unreacted alkaline phosphatase labeled IgE antibodies by a cation exchange chromatography.
Figure 19 is a chart illustrating a separation of rice allergen-FITC labeled IgE antibody complexes from unreacted alkaline phosphatase labeled IgE antibodies by a cation exchange chromatography.
Figure 20 is a chart illustrating a separation of wheat allergen-FITC labeled IgE antibody complexes from unreacted alkaline phosphatase labeled IgE antibodies by a cation exchange chromatography.
Figure 21 is a chart illustrating a separation of buckwheat allergen-FITC labeled IgE antibody complexes from unreacted alkaline phosphatase labeled IgE antibodies by a cation exchange chromatography.
Figure 22 is a chart illustrating a separation of soybean allergen-FITC labeled IgE antibody complexes from unreacted alkaline phosphatase labeled IgE antibodies by a cation exchange chromatography.
Figure 23 is a standard curve exhibiting quantitative changes of fluorescent strengthes derived from (DNP-BSA)-FITC labeled IgE antibody complexes when continuously applied to a column.
Figure 24 is a chart illustrating a separation of (DNP-BSA)-FITC labeled IgE antibody complexes from unreacted FITC labeled IgE antibodies by an anion exchange chromatography.

### BEST MODE FOR CARRYING OUT THE INVENTION

The present invention will be described in detail hereinafter.

The sample which may be assayed in the method of the present invention is not particularly limited, as long as there is a possibility that it contains an allergen. Examples thereof are almost all foods likely to be eaten by patients suffering from an allergy, for example, dairy products (such as milk, cheese, or yogurt), eggs (such as a hen's egg), grain (such as wheat or rice), beans (such as a soybean), vegetables, fruit, fish, seaweeds, meat, or processed food thereof; pollen (such as the pollen of cedar, rice, or ragweed); medicines (such as vaccines or penicillin); fur of animals (such as the fur of a dog or cat); ticks (such as Dermatophagoides farinae or Dermatophagoides pteronyssinus); insects (such as a midge); mold (such as Candida); fiber materials (such as silk); or dust in a room. The method of the present invention is particularly suitable for assaying foods.

A liquid sample may be used as the sample without any treatment, or after dilution or extraction with an appropriate liquid, for example, water, physiological saline, or a buffer. A solid sample may be used after dilution or extraction with an appropriate liquid, for example, water, physiological saline, or a buffer.

The allergen to be examined in the method of the present invention is not limited, as long as it is an allergen which causes allergosis. For example, there may be mentioned proteins contained in various foods, such as β-lactoglobulin or ovomucoid. Proteins which are not isolated as an allergen may be examined in the form of a food extract.

In the method of the present invention, an antibody specific to the allergen to be examined is first prepared. The immunoglobulin class of the antibody specific to the allergen to be examined is not limited but, for example, IgG, IgE, IgA, IgD, or IgM, preferably IgG or IgE, may be used. From the viewpoint of an ease of preparation of the antibody, IgG is preferable, and from the viewpoint of a greater sensitivity of the allergy reaction, IgE is preferable.

The antibody specific to the allergen to be examined may be prepared in accordance with a known appropriate method. For example, an antiserum may be taken from an immunized animal (particularly, a mammal, such as a rat, mouse or rabbit) after immunizing the animal by parenterally administering the allergen to be examined, as an immunogen to the animal. Further, a monoclonal antibody may be prepared by taking spleen cells from the immunized animal, and then fusing the cells with myeloma cells. Furthermore, a protein extract from a material (such as foods or pollen) causing allergies may be used as an immunogen to prepare an antiserum, and then an anti-wheat allergen antibody or anti-cedar pollen allergen antibody may be prepared therefrom.

The resulting antibody may be labeled by known methods. The label which may be used in the present invention is not particularly limited. For example, a fluorescent compound, enzyme or radioactive material may be used.

In a fluorescent assay wherein the fluorescent compound is used as a label, only a light with an excited wave length need be applied to the sample, and therefore, the assaying can be completed in a shorter time. An emission analysis using the enzyme has a superior sensitivity. An assay using the radioactive material has a superior assaying time and sensitivity. Accordingly, in the method of the present invention, the label varies with the object of the assay or a quantitative limitation.

In the method of the present invention, fluorescent compounds generally used in an immunological assay may be used. As a compound which may be used as a label by binding to an amino group of a protein, there may be mentioned, for example, fluorescein isothiocyanate (hereinafter sometimes referred to as FITC), tetramethylrhodamine isothiocyanate (TMRITC), dansyl chloride, rhodamine isothiocyanate, fluorescamine, chloronitrobenzoxadiazole or the like. As a compound which may be used as a label by binding to a thiol group of a protein, there may be mentioned (iodoacetylaminoethyl)nephthylamine sulfuric acid (AEANS), difluorescein cystine, fluorescein mercury acetic acid, or S-mercury-N-dansyl cystein.

As the enzyme, those for emission which are generally used in an immunological assay may be used. The enzyme is used in combination with a substrate and a coloring compound in a known manner. As the enzyme, there may be mentioned, for example, alkaline phosphatase, β-D-galactosidase, horseradish peroxidase, or catechol-o-methyl transferase.

As the radioactive material, those generally used in an immunological assay may be used. Examples of the radioactive material are I¹²⁵ or I¹³¹.

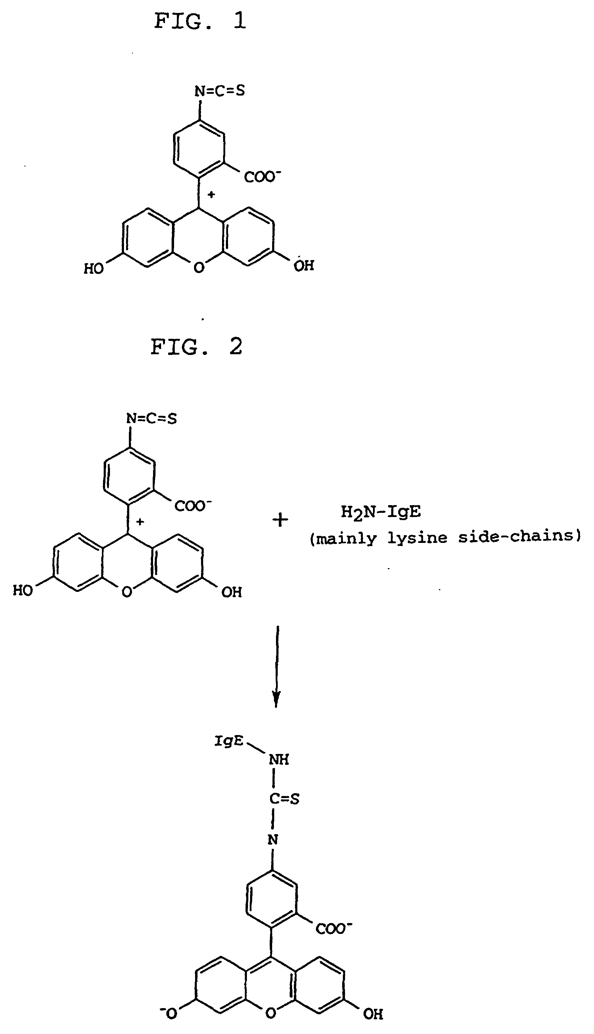

Of the above fluorescent compounds and enzymes for emission, fluorescein isothiocyanate (FITC) is particularly preferable because it is stable, does not inhibit an activity of the antibody, is easily labeled, has a good solubility, and has a spectroscopically advantageous property; namely, the maximum absorption wavelength is widely separated from the maximum fluorescent wavelength. The structure of FITC is shown in Fig. 1.

The above labels can be bound to the antibody by known appropriate methods.

For example, when FITC is reacted with an antibody (protein) under an alkaline condition, isothiocyanate groups in FITC are reacted with amino groups in the antibody, mainly, amino groups in lysine groups, to form stable dicarbamide bonds. The reaction scheme is shown in Fig. 2.

Therefore, when FITC is used as the fluorescent label, incubation may be carried out under an alkaline condition, preferably a pH of 8 to 9, at room temperature or less, preferably 15 to 25^{°}C, for 2 hours or more, preferably 2 to 8 hours, or at about 4^{°}C, overnight or more, preferably for 8 to 20 hours, so that a molar ratio of FITC and the antibody becomes preferably 3 to 5:1.

The labeling of the antibody with the enzyme for emission can be carried out by known appropriate methods. For example, a method for modifying the antibody by reducing hydroxy groups in sugar chains of the enzyme and forming Schiff bases with amino groups in the antibody, or a method wherein disulfide bonds in the hinge region of the antibody are reduced and the amino groups in the enzyme then bonded to or cross-linked with the produced thiol groups, are known. In the above methods it is preferable to use a cross-linking agent, because a steric hindrance between the enzyme for emission and the antibody is reduced, and a dispersion of a bonding molar ratio of the antibody and the enzyme for emission is lowered. The cross-linking agent is not particularly limited but, for example, N-succinimidyl-3-(2-pyridyldithio)propionate (SPDP) may be used.

The labeling with the radioactive material can be carried out by known appropriate methods, such as a chloramine T method. More particularly, the antibody is generally dissolved in a buffer having a pH of 7 and a radioactive iodine is added thereto. To the solution obtained is added a solution of chloramine T (N-chloro-p-toluenesulfonamide) (500 µg/ml) at an amount of 10 mg of chloramine T per 1 mg of the antibody, and a radioactive-iodization is performed for 10 minutes. Sodium thiosulfate (10 µg/1 mg of the antibody) is then added to halt the reaction, and any unreacted radioactive iodine is then removed by dialysis.

The labeled antibodies are then separated from the unreacted labels and purified. The separating and purifying methods used may be any appropriate method, such as dialysis or gel permeation column chromatography.

For example, the antibody labeled with FITC can be purified, by treating the reaction liquor containing the FITC labeled antibodies after incubation, by a dialysis with a buffer having a neutral pH and thereabout to an alkaline pH, preferably a pH of 6 to 9, to remove the unreacted FITC. Alternatively, the FITC labeled antibodies can be separated from the unreacted FITC by a gel permeation column chromatography. When the separation is carried out by a gel permeation column chromatography, a cross-linking carrier having a pore size such that the FITC (molecular weight = 389.4) and the antibody (molecular weight of IgE = about 180,000; molecular weight of IgG = about 150,000) can be separated, may be used as a gel permeation carrier. The cross-linking carrier may be, for example, dextran polymer, agarose, polyacrylamide, or a mixture of agarose and polyacrylamide. As a mobile phase, a neutral or alkaline buffer, preferably a buffer having a pH of 7 to 8, for example, malonate, phosphate, carbonate, citrate-sodium hydroxide, acetate, borate-sodium hydroxide, glycine-sodium hydroxide, tris-hydrochloride buffer, or the like may be used. The FITC labeled antibodies can be purified by measuring absorptions of the elute from the gel permeation column chromatography at 280 nm in an ultraviolet absorption derived from the antibody, and at 520 nm in a fluorescence absorption derived from the FITC, and taking the fraction of which the peaks overlap each other.

The FITC labeled antibody prepared by purification by a dialysis or gel permeation column chromatography does not have a constant binding molar ratio (FITC/antibody) of the FITC and the antibody but is a mixture of the labeled antibodies with various binding molar ratios. Therefore, after the above purification by a dialysis or gel permeation column chromatography, it is preferable to fractionate the purified product by an anion exchange chromatography and thereby obtain a product having a constant binding molar ratio (FITC/antibody) of the FITC and the antibody. When the labeled antibody having a constant binding molar ratio is used, more reliable data can be obtained in comparison with data obtained from the mixture of the labeled antibodies with various binding molar ratios. The binding molar ratio per se is not particularly limited, but is preferably 1 to 2.

Examples of an anion exchange carrier which may be used are a weak anion exchange carrier containing agarose beads as a carrier, a carrier having diethylaminoethyl (DEAE) groups, a carrier having cellulose gel as a carrier, or a carrier having dextran as a carrier. As a mobile phase, a neutral or weak alkaline buffer, preferably a buffer having a pH of 7 to 8, for example, malonate, phosphate, carbonate, citrate-sodium hydroxide, acetate, borate-sodium hydroxide, glycine-sodium hydroxide, tris-hydrochloride buffer, or the like may be used.

The separation of the antibody labeled with the enzyme from the unreacted enzyme, or the antibody labeled with the radioactive material from the unreacted radioactive material, may be carried out by any appropriate method, such as a dialysis or gel permeation column chromatography.

In the method of the present invention, the next step comprises bringing the sample possibly containing the allergen to be examined into contact with the labeled antibody.

As mentioned, the sample possibly containing the allergen to be examined denotes various materials which a patient suffering from an allergy may encounter, particularly foods eaten by a patient suffering from an allergy, for example, dairy products, eggs, or wheat, or processed foods thereof. The sample is brought into contact with the labeled antibody in, for example, a weak acidic to weak alkaline liquid, preferably a liquid having a pH of 5 to 9. For example, when solid wheat is examined, it is preferable to prepare the sample by treating it with a 0.5 M (or less) saline solution to solubilize proteins. When the liquid to be examined is fermented milk, it is preferable to prepare the sample by adjusting the pH.

The sample is brought into contact with the labeled antibody under the condition that an antigen-antibody reaction between the allergen to be examined in the sample and the labeled antibody can be carried out. For example, the sample and the labeled antibody may be incubated under a weak acidic to a weak alkaline condition, preferably a pH of 5 to 9, at 37^{°}C or less, preferably 25 to 37^{°}C, for 20 minutes or more, preferably 20 to 30 minutes. Particularly, conditions such that the antibody or enzyme in the labeled antibody is not deactivated, and the label is not liberated should be selected.

For example, when the purified FITC labeled antibody is used, a mixture of the FITC labeled antibody and the sample are incubated at 37^{°}C or less, preferably 25 to 37^{°}C, for 20 minutes, preferably 20 to 30 minutes, so that the antibody is not deactivated.

When the sample is brought into contact with the labeled antibody, for example by incubation as mentioned above, a complex of the allergen and the labeled antibody is formed in a reaction liquor, in proportion to an amount of the allergen present in the sample, if the allergen to be examined is present therein.

In the method of the present invention, the subsequent step is a treatment of the resulting reaction liquor by ion exchange chromatography, to separate the allergen labeled antibody complex and the unreacted labeled antibody.

The ion exchange chromatography which may be used in the present invention is not particularly limited. An ion exchanger which can adsorb the unreacted labeled antibody, in relation to an isoelectric point of the labeled antibody protein used and the pH value of the buffer as the mobile phase, should be selected. For example, when the isoelectric point of the labeled antibody used is 7, and the pH value of the buffer used as the mobile phase is 5, the labeled antibody is positively charged. Therefore, a cation exchanger is used. When a cation exchanger is used, it is necessary to use a buffer having a pH of more than 7 as the mobile phase, and to give the labeled antibody a negative charge. Further, when the label is the enzyme, consideration is needed to ensure that the ion exchanger does not adsorb the labeled antibody at the side of the enzyme, particularly taking into account the isoelectric point of the enzyme with which the antibody is labeled. Generally, an antibody is not resistant to alkali. Therefore, it is preferable to use a cation exchanger using an acidic mobile phase, to thus maintain the activity of the antibody. It is possible to adjust an optimum pH value of the buffer used as the mobile phase, taking into account the combination of the isoelectric point of the labeled antibody and an exchangeable pH range of the ion exchanger. In this case, it is necessary to ensure that the conditions are such that deactivation of the antibody or the label, liberation of the label from the antibody, and/or dissociation of the allergen-antibody complex does not occur at the pH value of the buffer. Further, the condition that the allergen-antibody complex is not adsorbed to the ion exchanger in the range where the unreacted labeled antibody is adsorbed to the ion exchanger must be selected. When the antibody is bound to the allergen to form the complex, the charge of the antibody is changed, and thus the difference in the electrostatic force between the antibody and the ion exchanger becomes smaller, or the charge of the antibody is reversed. Therefore, the adsorbability between the complex and the ion exchanger becomes lower than that between the labeled antibody and the ion exchanger, and thus it is possible to adsorb the labeled antibody to the ion exchange and elute only the complex.

A cation-exchange carrier which may be used in the present invention is not particularly limited, but may be, for example, prepared by introducing a cation exchanging group of various functional groups, such as a carboxymethyl, sulfomethyl, sulfoethyl, or phosphoric acid group, into a carrier, such as an agarose bead, cellulose gel, or dextran. As a mobile phase, a weak acidic buffer, preferably a buffer having a pH of 4.5 to 6, for example, malonate, phosphate, carbonate, citrate-sodium hydroxide, or acetate buffer, or the like may be used.

The anion exchanger which may be used in the present invention is not particularly limited, but may be prepared by introducing an anion exchanging group, such as an aminoethyl, diethylaminoethyl or quanidinoethyl group, into the above carrier.

When the reaction liquor obtained in the above contacting step is treated by the ion exchange chromatography, only the allergen labeled antibody complex is eluted. The unreacted labeled antibody is bound to the carrier in the ion exchanger and not eluted. Therefore, the complex of the allergen to be examined and the labeled antibody, i.e., the complex of the allergen to be examined and the antibody labeled with the fluorescent material, the complex of the allergen to be examined and the antibody labeled with the enzyme for emission, or the complex of the allergen to be examined and the antibody labeled with the radioactive material, are easily separated from the unreacted labeled antibody.

The unreacted labeled antibody which is bound to the carrier can be eluted with an aqueous solution, such as a sodium chloride aqueous solution containing a salt at a concentration of 0.2 M or more, for example, 0.2 to 1 M. Therefore, a cycle comprising an addition of the sample, the elution of the complex of the allergen to be examined and the labeled antibody, i.e., the subject material of the assay, the elution of the unreacted labeled antibody, i.e., non-subject material of the assay, and an equilibrating of the column by the passage of a weak acidic buffer can be successively repeated for a column. Therefore, the sample can be successively applied to the column and analyzed.

Within the ion-exchangeable capacity of the ion exchange column, it is not necessary to elute the unreacted labeled antibody, i.e., non-subject material of the assay, and equilibrate the column by the passage of the weak acidic buffer. Therefore, it is possible to successively apply subsequent samples after the complex of the allergen to be examined and the labeled antibody, i.e., the subject material of the assay, is eluted from the previous sample within the ion-exchangeable capacity of the ion exchange column. In this case, the elution of the unreacted labeled antibody, i.e., non-subject material of the assay, and the equilibrating of the column by the passage of the weak acidic buffer can be omitted, and a greater operating efficiency can be attained.

In the method of the present invention, signals from the labels contained in the complex of the allergen to be examined and the labeled antibody, namely, fluorescence from the fluorescent compound label, emission from the enzyme for coloring, or radioactivity from the radioactive material is detected from the elute which is eluted from the ion exchange chromatography and contains the complex of the allergen to be examined and the labeled antibody. A standard curve can be obtained by carrying out the same procedure except that a known amount of the allergen is used. Further, it is possible to detect the presence of the allergen in the sample.

The fluorescence from the fluorescence compound label can be detected or measured by any known method, but preferably an automatic analysing method, using any known apparatuses, such as a fluoro-spectrophotometer.

The emission from the enzyme label can be detected or measured by adding an appropriate substrate and detecting or measuring a light having a wavelength corresponding to the substrate, by any known method, but preferably an automatic analysing method, using any known apparatuses, such as a luminescence reader, i.e., an emission analyzer. When an alkaline phosphatase is used as the enzyme for emission, the substrate used may be, for example, fluorescein diphosphori acid, 4-methylumbelli feryl phosphoric acid, 3-(2'-spiroadamantane)-4-methoxy-4-(3''-phosphoryloxy)phenyl-1,2-dioxycetane (AMPPD), D-luciferin-o-phosphate (LUCP), or o-aminophthalylhydrazide-o-phosphate (luminal-o-phosphate; LUMP). When β-D-galactosidase is used as the enzyme for emission, the substrate used may be, for example, 3-(2'-spiroadamantane)-4-methoxy-4-(3"-β-D-galactopyranosyloxy)-phenyl-1,2-dioxycetane (AMPGD) or o-aminophthalylhydrazide-β-D-galactoside (luminol-β-D-galactoside; LUMG). When horseradish peroxidase is used as the enzyme for emission, the substrate used may be, for example, homovanillic acid, tyramine, p-cresol, p-hydroxyphenylacetic acid, or p-hydroxyphenylpropionic acid (HPPA). When a catechol-o-methyl transferase is used as the enzyme for emission, the substrate used may be, for example, 2-(3,4-dihydroxyphenyl)naphtho[1,2-d]thiazole (DNT).

The radioactivity from the radioactive material label can be measured by a scintillation counter or the like.

A plurality of samples may be treated batchwise, for example, by
(1) first carrying out, for all the samples to be examined, the step of bringing the sample into contact with the labeled antibody, and
(2) carrying out, for all the samples to be examined, the step of removing the unreacted labeled antibody by ion exchange chromatography, and
(3) carrying out, for all the samples to be examined, the step of detecting the signals from the allergen labeled antibody complex.

Alternatively, a plurality of samples may be successively treated so that an analyzing procedure is successively commenced on each of the plurality of samples after an appropriate interval, and each sample is successively subjected to the above contacting step (1), the above removing step (2) and the above detecting step (3), respectively. Namely, a first sample is continuously treated by the contacting step (1), the removing step (2), and the detecting step (3), and after an appropriate interval from the start of the analyzing procedure of the first sample, an analyzing procedure of a second sample is commenced, and the above contacting step (1), the above removing step (2) and the above detecting step (3) are carried out for the second sample, respectively. The above continuous process can shorten a reaction time and a waiting period for measuring, and make easier the assay of the allergen automatic.

### EXAMPLE

The present invention now will be further illustrated by, but is by no means limited to, the following examples.

### Example 1: Preparation of complex of allergen and labeled antibody

### (1) Preparation of anti-DNP-IgE antibody labeled with FITC

To 15 ml of a 0.5 M carbonate buffer (pH 9) were added 10 mg of FITC (fluorescein isothiocyanate), and 50 mg of an anti-DNP (dinitrophenyl) IgE antibody (Seikagaku-Kogyo; liquid), and the whole was incubated at room temperature for 4 hours. Thereafter, 15 ml of the resulting liquid was dialyzed at 4^{°}C for 72 hours in 3 liter of a 0.01 M carbonate buffer (pH 9), to remove unreacted FITC.

### (2) Contact with sample

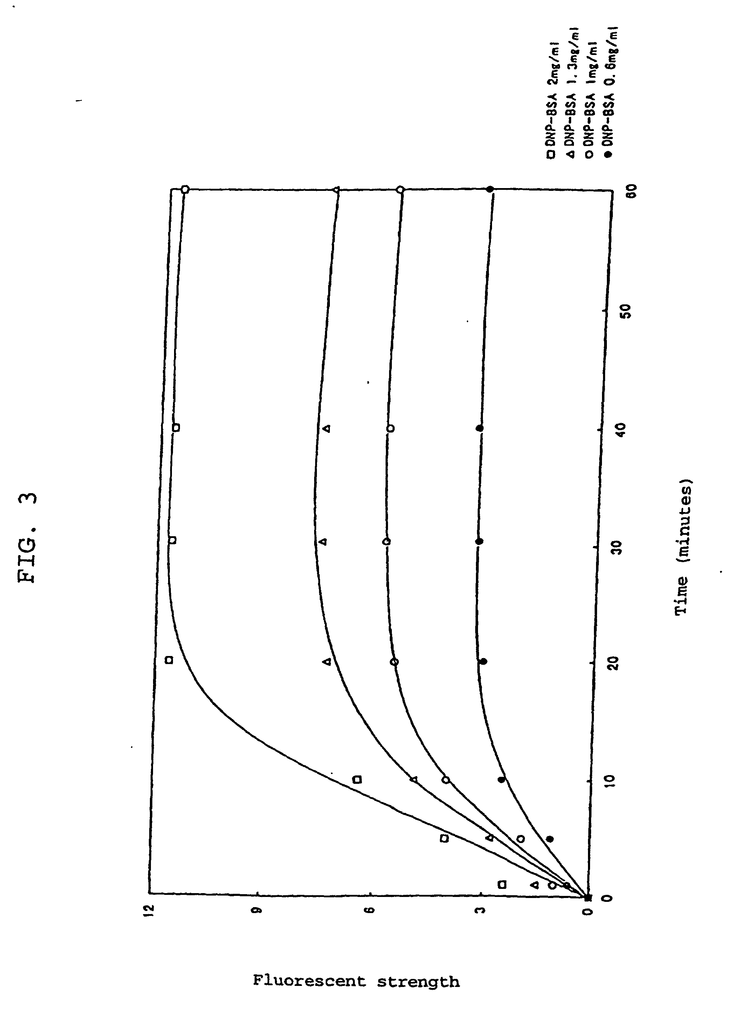

After 100 µl of the 1.8 ng/µl FITC labeled IgE antibody prepared in Example 1(1) [in a 0.01 M carbonate buffer (pH 9)] was mixed with 100 µl of a model allergen, i.e., DNP-BSA (dinitrophenylated bovine serum albumin) [in a 0.01 M carbonate buffer (pH 9)], the whole was incubated at 37^{°}C. The incubation (reaction) time was 1, 5, 10, 20, 30, 40 or 60 minutes, and the concentration of the model allergen, DNP-BSA was 0.6 mg/ml, 1.0 mg/ml, 1.3 mg/ml, or 2.0 mg/ml. The amount of the allergen labeled antibody complex formed with time was determined, and the results are shown in Fig. 3. In Fig.3, □ shows the results of 2 mg/ml DNP-BSA, Δshows the results of 1.3 mg/ml DNP-BSA, ○ shows the results of 1 mg/ml DNP-BSA, and ● shows the results of 0.6 mg/ml DNP-BSA. It is apparent from Fig. 3 that the fluorescent strength becomes constant at each DNP-BSA concentration when the incubation (reaction) time is 20 minutes or more.

### (3) Fractionation of allergen labeled antibody complex by ion-exchange chromatography

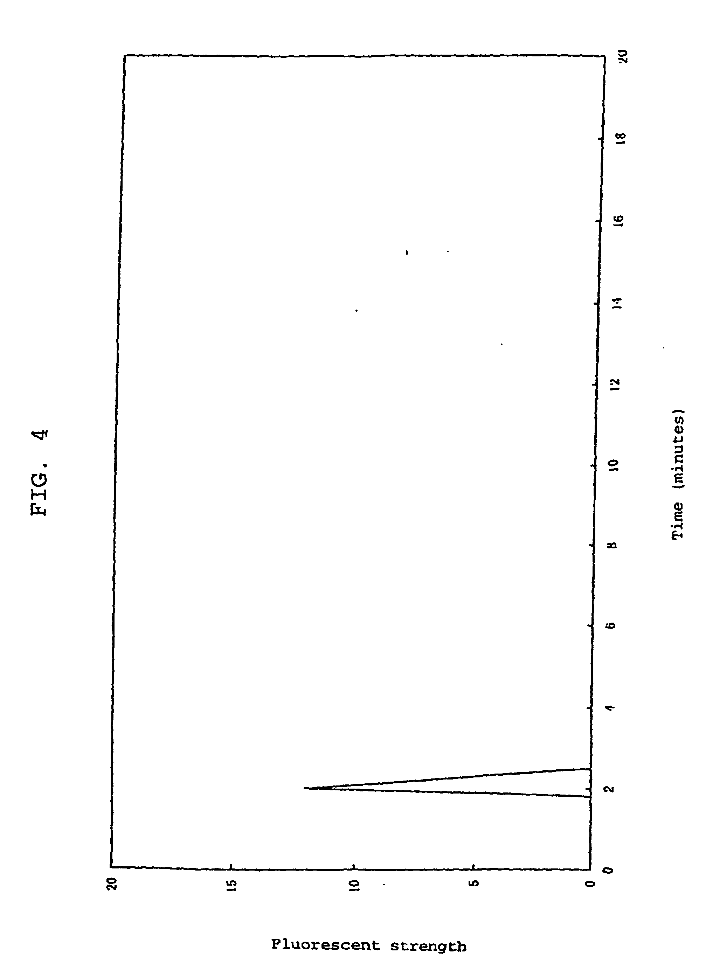

To a cation-exchange column (HITRAP™SP-Sepharose Fast Flow; diameter = 16 x 25 mm; manufactured by Pharmacia), 200 µl of the reaction liquid (incubation time = 20 minutes; concentration of DNP-BSA = 2 mg/ml) prepared in Example 1(2) was added. As a mobile phase, a 50 mM malonate buffer (pH 5.0) was used at a flow rate of 1 ml/min. A fluorescent-spectrophotometer (F-2000; manufactured by Hitachi Ltd.) was used to conduct a detection by measuring a fluorescent strength stemming from the FITC. The results are shown in Fig. 4.

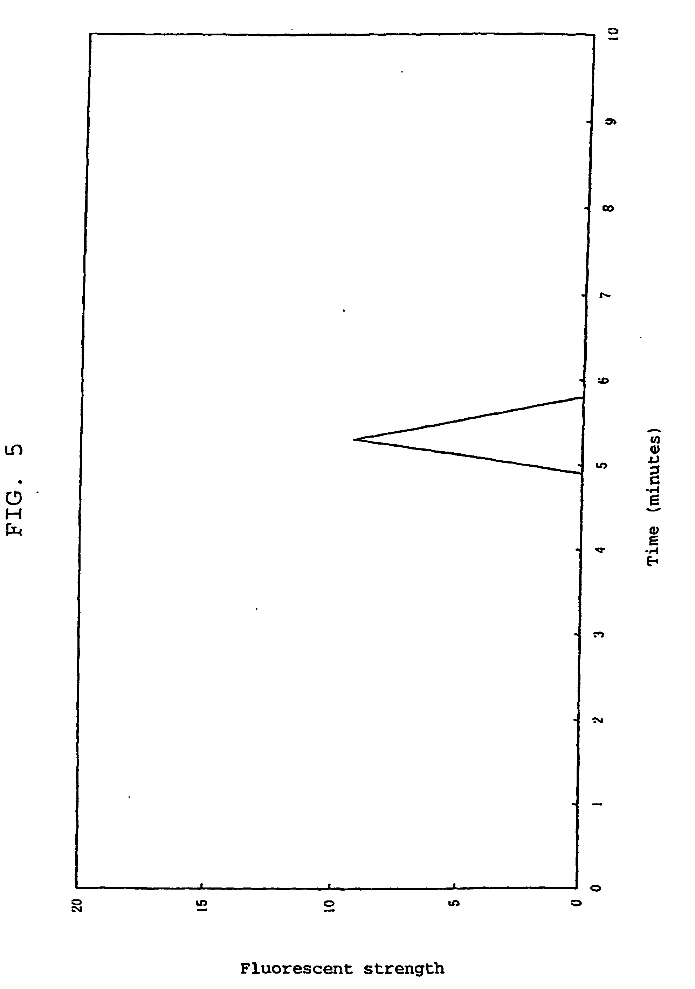

As apparent from Fig. 4, only the allergen labeled antibody complex was eluted in the malonate buffer eluting fraction. After the allergen labeled antibody complex was eluted, a 0.5 M NaCl aqueous solution was passed through the column, and as shown in Fig. 5, the unreacted labeled IgE antibody was eluted.

### Example 2: Reaction specificity of anti-DNP-IgE antibody labeled with FITC

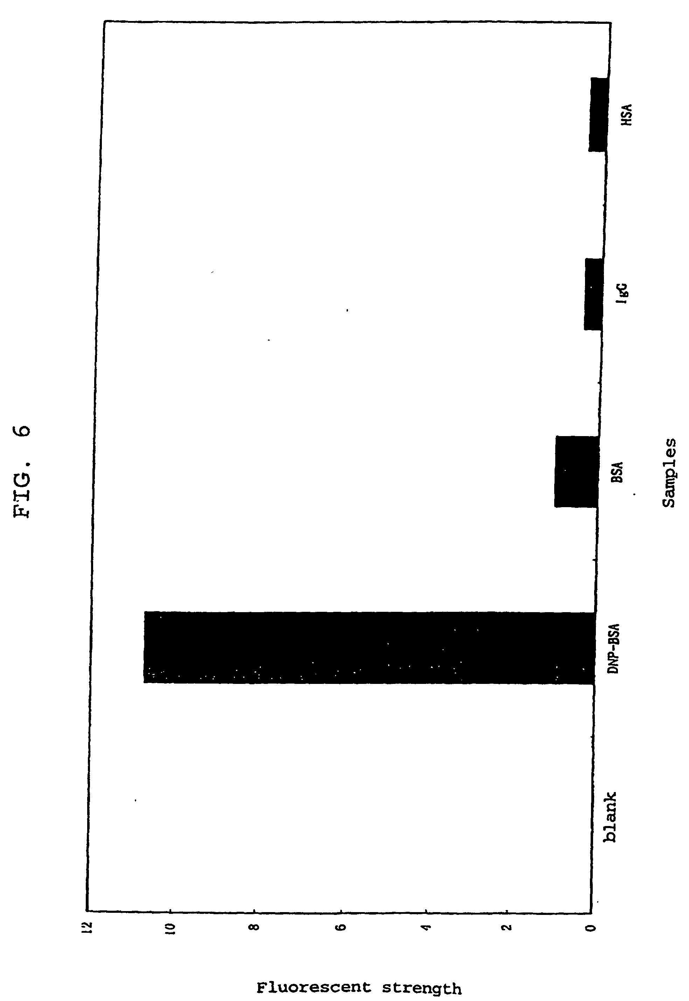

In the present Example, a reaction specificity of an anti-DNP-IgE antibody labeled with FITC was evaluated. As an antigen sample, DNP-BSA used in Example 1, BSA (manufactured by SIGMA; powder), IgG (manufactured by SIGMA; liquid), and HSA (human serum albumin; manufactured by Seikagaku Kogyo; powder) were used. The antigen sample was incubated with the FITC labeled IgE antibody prepared in Example 1(1) at 37^{°}C for 20 minutes. Thereafter, the whole was applied to the ion exchange column used in Example 1(3), and the fluorescent strength from the antigen sample labeled antibody complex eluted from the malonate buffer used in Example 1(3) was measured. A control test (blank) was carried out wherein a phosphate buffered physiological saline was used instead of the above antigen samples. The results are shown in Fig.6. No increase of the fluorescent strength was observed in BSA, IgG, and HSA, whereas an increase of the fluorescent strength was observed in DNP-BSA. Therefore, it is apparent that an allergen can be selectively assayed by the method in accordance with the present invention.

### Example 3: Quantifiability

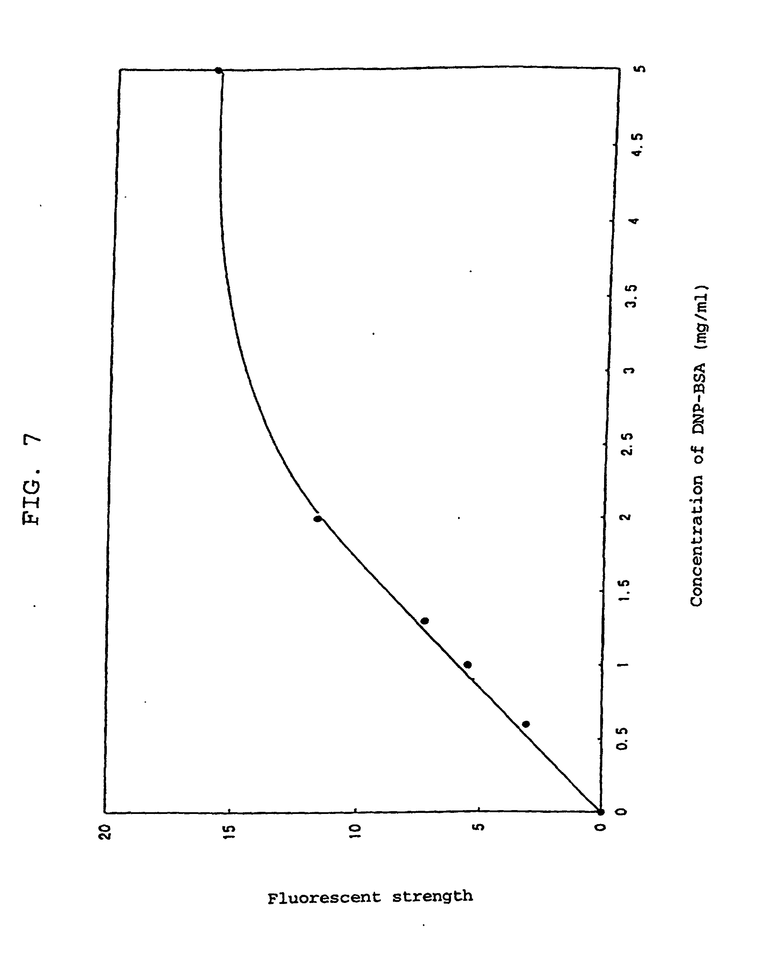

To evaluate the quantifiability (capability of quantitative determination) and the quantitative scope of the method of the present invention, a sample liquid containing DNP-BSA as a model allergen in a concentration of 0, 0.6, 1.0, 1.3, 2.0, 5.0 mg/ml in a 0.05 M malonate buffer (pH 5) was prepared. After 100 µl of the DNP-BSA solution and 100 µl of the 1.8 ng/µl FITC labeled IgE antibody liquid used in Example 1(1) were mixed, the mixture was incubated at 37^{°}C for 20 minutes. The reaction liquid was treated by the same procedure as in Example 1(3), and the fluorescent strength from the antigen sample labeled antibody complex eluted was measured. The results are shown in Fig.7. A linear correlation was observed where the allergen concentration was 0 to 2 mg/ml.

### Example 4: Assay of wheat allergen with alkaline phosphatase labeled IgE antibody

### (1) Preparation of wheat allergen extract

Wheat flour (hakuriki-ko) (10 g) was washed with 100 ml of diethyl ether, and centrifuged at 2000 x g for 3 minutes. The washing and centrifuging procedures were repeated 3 times. To the whole precipitate, 100 ml of a 0.1 M phosphate-buffered physiological saline (pH 7.4) was added, and the whole was stirred for 2 hours. Then, the supernatant was dialyzed in 3 liters of a 0.05 M phosphate-buffered physiological saline (pH 7.4) at 4^{°}C for 72 hours to obtain a wheat allergen extract (35 µg/ml).

### (2) Preparation of alkaline phosphatase labeled IgE antibody

According to a conventional manner, 2 ml of an anti-wheat allergen antiserum was obtained from a rat immunized with the wheat allergen extract prepared in Example 4(1). To the antiserum, sodium sulfate was added until its concentration became 18 w/v %. The resulting precipitate was dialyzed in 2 liters of a 0.01 M phosphate buffer (pH 8.0) for 48 hours. Thereafter, the resulting dialyzed solution was added to a DEAE weak basic anion-exchange column (5 ml; Hitrap column; manufactured by Pharmacia), and eluted with a phosphate buffer (pH 8) containing 0.15 M sodium chloride with a linear concentration gradient of 0.01 M to 0.2 M phosphoric acid to obtain an anti-wheat allergen-IgE antibody fraction.

The resulting anti-wheat allergen-IgE antibody fraction was labeled with alkaline phosphatase by the following method:

Alkaline phosphatase (manufactured by Sigma) (0.45 mg) was dissolved in 1 ml of a 0.1 mM tris-hydrochloride buffer (pH 7.0) containing 0.1 mM-MgCl₂ and 0.1 mM-ZnCl₂. To 1 ml of the solution was added 0.5 mg of a cross-linking agent, i.e., N-succinimidyl-3-(2-pyridylthio)propionate (hereinafter referred to SPDP; manufactured by Pharmacia), and the whole was incubated at room temperature for 2 hours. Thereafter, the unreacted SPDP was removed by a desalting column to obtain SPDP-bound alkaline phosphatase. Then 1 ml of an anti-wheat allergen-IgE antibody fraction (1.1 mg/ml) wherein disulfide bonds in the hinge region had been reduced with dithiothreitol (DTT) was mixed with 1 ml of the above SPDP-bound alkaline phosphatase, and the whole was incubated at 4^{°}C for 12 hours to thereby cross-link the amino groups in the alkaline phosphatase and the thiol groups in the anti-wheat allergen-IgE antibody with SPDP. The resulting reaction liquor was added to a gel permeation column to separate the alkaline phosphatase labeled anti-wheat allergen-IgE antibody from the unreacted IgE antibody or the unreacted SPDP-bound alkaline phosphatase, and obtain an alkaline phosphatase labeled anti-wheat allergen-IgE antibody fraction (7 µg/ml).

### (3) Contact with a sample

The wheat allergen extract (35 µg/ml; 50 µl) prepared in Example 4(1) was mixed with 50 µl of the alkaline phosphatase labeled anti-wheat allergen-IgE antibody prepared in Example 4(2), and the whole was incubated at 37°C for 20 minutes to cause an antigen-antibody reaction.

### (4) Fractionation of allergen labeled antibody complex by ion-exchange chromatography

After the antigen-antibody reaction was completed, 100 µl of the mixture was added to the cation-exchange column used in Example 1(3). The column was eluted with a 50 mM malonate buffer (pH 5.0) as a mobile phase for 2 minutes at a flow rate of 1 ml/min, and then a sodium chloride aqueous solution with a linear concentration gradient of 0 to 0.15 M was passed through. The eluted solutions were divided into fractions of 0.25 ml.

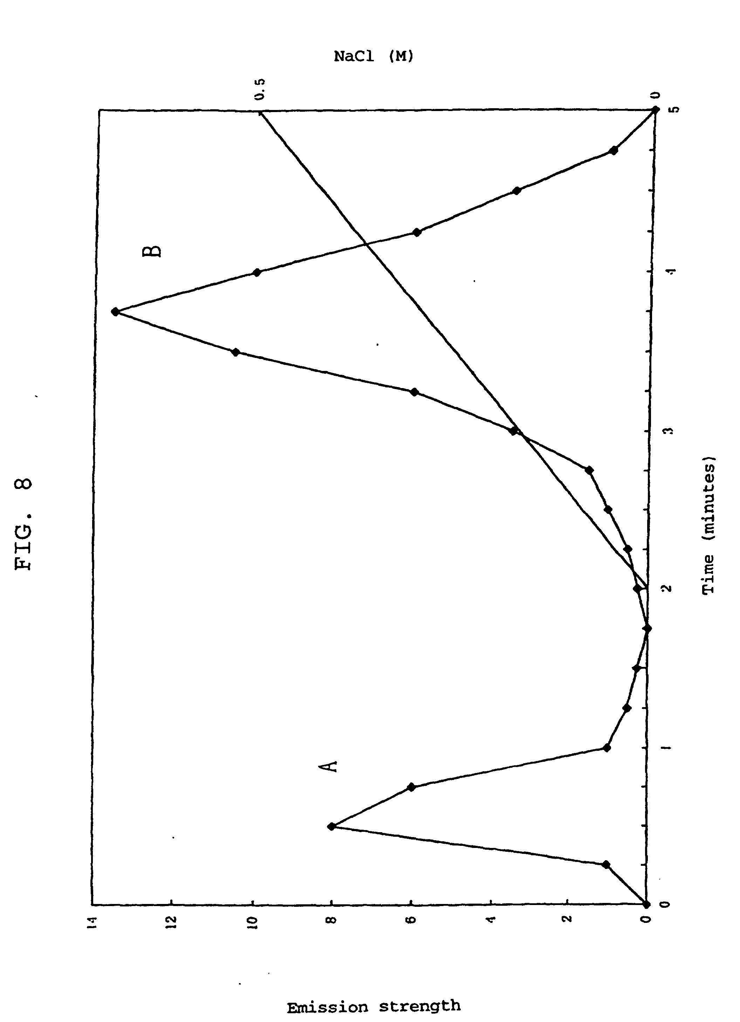

For analyzing, 300 µl of 3-(2'-spiroadamantane)-4-methoxy-4-(3''-phosphoryloxy)phenyl-1,2-dioxycetane (AMPPD) as a substrate for emission was added, and the whole was incubated at 37^{°}C for 15 minutes. Thereafter, an emission strength was measured by a luminescence (reader) (BLR301; Aloka). The results are shown in Fig. 8. It is apparent from Fig. 8 that only the allergen labeled antibody complex (peak A) was eluted in the malonate buffer eluting fraction, and that the unreacted alkaline phosphatase labeled anti-wheat allergen-IgE antibody (peak B) was eluted after the passage of the NaCl aqueous solution with the linear concentration gradient of 0 to 0.15 M. Namely, the product of the antigen-antibody reaction and the unreacted alkaline phosphatase labeled anti-wheat allergen-IgE antibody were properly separated. Therefore, it is apparent that an allergen can be assayed by the method in accordance with the present invention.

### Example 5: Evaluation of quantifiability

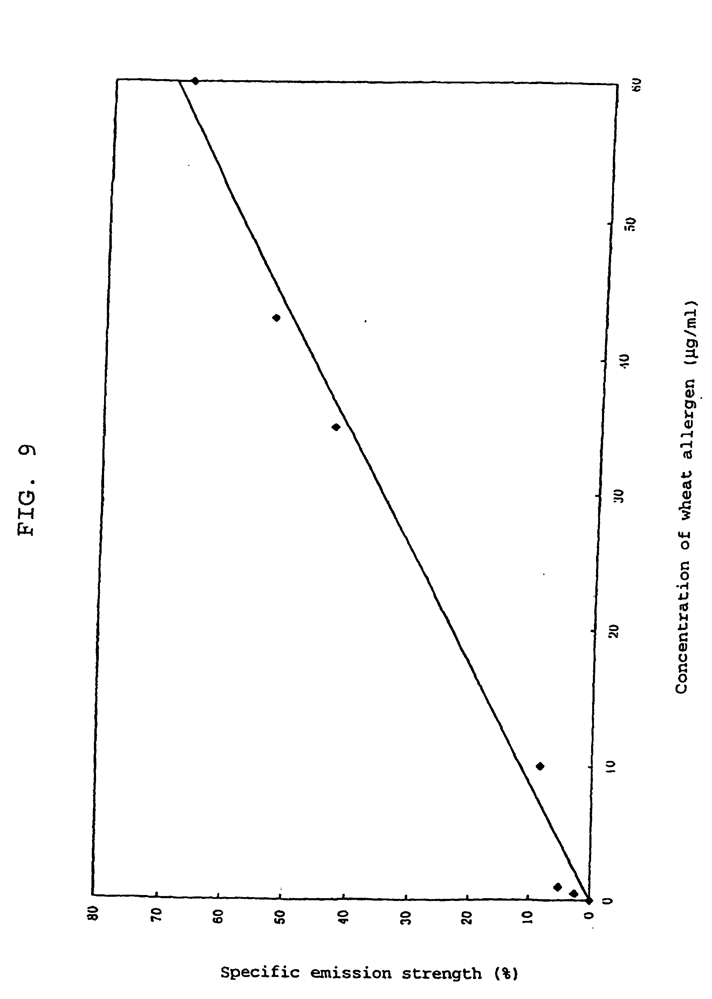

The quantifiability (capability of quantitative determination) was evaluated, by carrying out the procedures disclosed in Examples 4(3) and 4(4), using the known amounts of the wheat allergen and the alkaline phosphatase labeled anti-wheat allergen-IgE antibody of Example 4(2). The results are shown in Fig. 9. Fig. 9 shows a relative emission strength calculated to a 100% emission strength obtained by adding the whole sample into the column.

A linear relation was observed where the concentration of the wheat allergen was 0 to 60 µg/ml. As shown above, the wheat allergen was quantitatively determined in the method of the present invention.

### Example 6: Measurement of allergen by alkaline phosphatase labeled IgG antibody

### (1) Measurement of allergen by anti-β-lactoglobulin-IgG antibody

According to a conventional method, 2 ml of an anti-β-lactoglobulin antiserum was obtained from a rat immunized with a milk allergen, i.e., β-lactoglobulin (manufactured by SIGMA; powder; prepared by crystallization 3 times). The resulting antiserum was added to a protein A column to obtain an IgG antibody fraction. The procedure in Example 4(2) was repeated except that the resulting IgG antibody fraction was used, to thereby obtain an alkaline phosphatase labeled anti-β-lactoglobulin-IgG antibody.

50 µl of the resulting alkaline phosphatase labeled anti-β-lactoglobulin-IgG antibody (20 µg/ml: in a 0.05 M phosphate buffer) and 50 µl of β-lactoglobulin (30 µg/ml: in a 0.05 M phosphate buffer) were incubated at room temperature for 20 minutes to conduct an antigen-antibody reaction.

Separate therefrom, rice, wheat, buckwheat, or soybean was ground, and after a 3% sodium chloride aqueous solution was added, the ground product was homogenized, centrifuged, and further centrifuged, to remove sodium chloride, and then an extract was prepared. Further, albumen (the white of an egg) was diluted with a equal volume of water to prepare a diluted albumen. Then 50 µl of the resulting extract or diluted liquid (30 µg protein/ml) and 50 µl of the alkaline phosphatase labeled anti-β-lactoglobulin-IgG antibody (20 µg/ml) were incubated at room temperature for 20 minutes to carry out an antigen-antibody reaction.

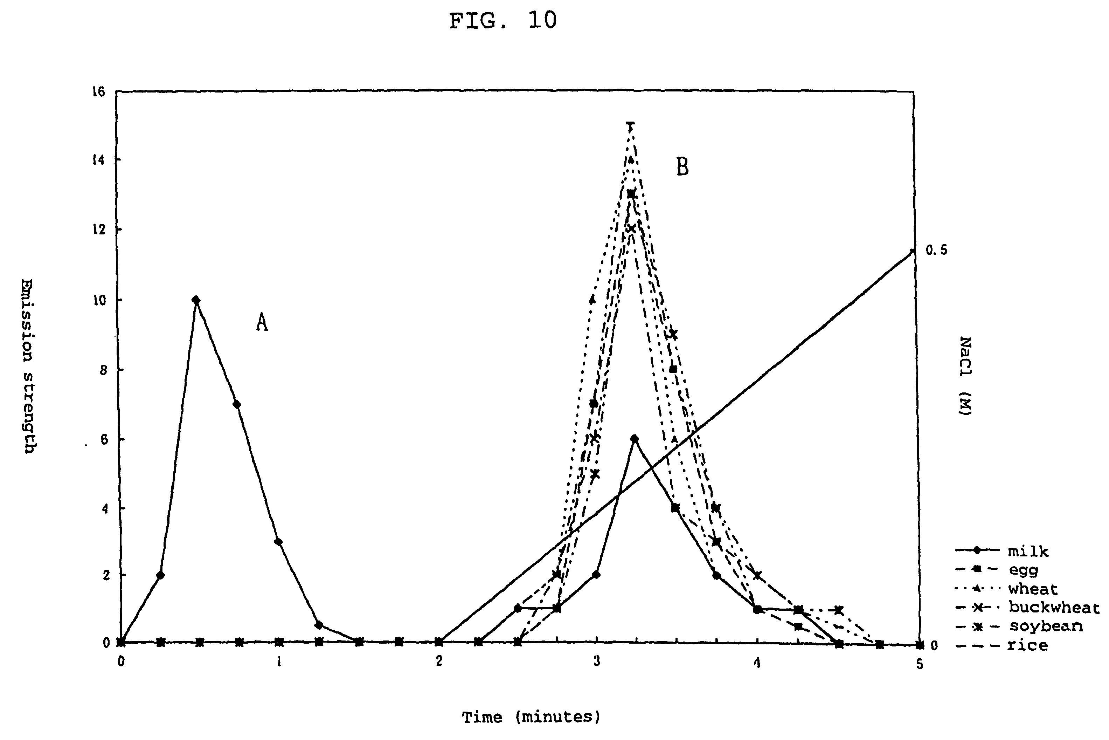

100 µl of the reaction liquor was fractionated in accordance with the procedure in Example 4(4), and the emission strength was measured. The results are shown in Fig. 10. In Fig.10, ◆ shows the results where the sample examined was the β-lactoglobulin solution (milk), ■ shows the results where the sample examined was the diluted albumen liquid, ▲ shows the results where the sample examined was the wheat extract, × shows the results where the sample examined was the buckwheat extract, * shows the results where the sample examined was the soybean extract, and - shows the results where the sample examined was the rice extract.

When the sample was the β-lactoglobulin solution (milk), only the allergen (β-lactoglobulin) labeled antibody complex (peak A) was eluted in the malonate buffer eluting fraction, and the unreacted labeled anti-β-lactoglobulin-IgG antibody (peak B) was eluted after the passage of the NaCl aqueous solution with the linear concentration gradient of 0 to 0.15 M. Further, when the sample was the diluted albumen liquid, the wheat extract, the buckwheat extract, the soybean extract, or the rice extract, only the unreacted labeled anti-β-lactoglobulin-IgG antibody was eluted. As above, the product from the antigen-antibody reaction and the unreacted labeled antibody were properly separated. and only the antigen-antibody was able to be analyzed.

### (2) Measurement of allergen by anti-albumen allergen-IgG antibody

The procedure of Example 6(1) was repeated except that the diluted albumen liquid prepared in Example 6(1) was used as an antigen instead of β-lactoglobulin, to thereby obtain an anti-albumen allergen-IgG antibody fraction. Then an alkaline phosphatase labeled-anti-albumen allergen-IgG antibody was prepared, using the resulting IgG antibody fraction. An antigen-antibody reaction was carried out with the diluted albumen liquid, the β-lactoglobulin solution, the wheat extract, the buckwheat extract, the soybean extract or the rice extract prepared in Example 6(1), and the antigen-antibody was analyzed.

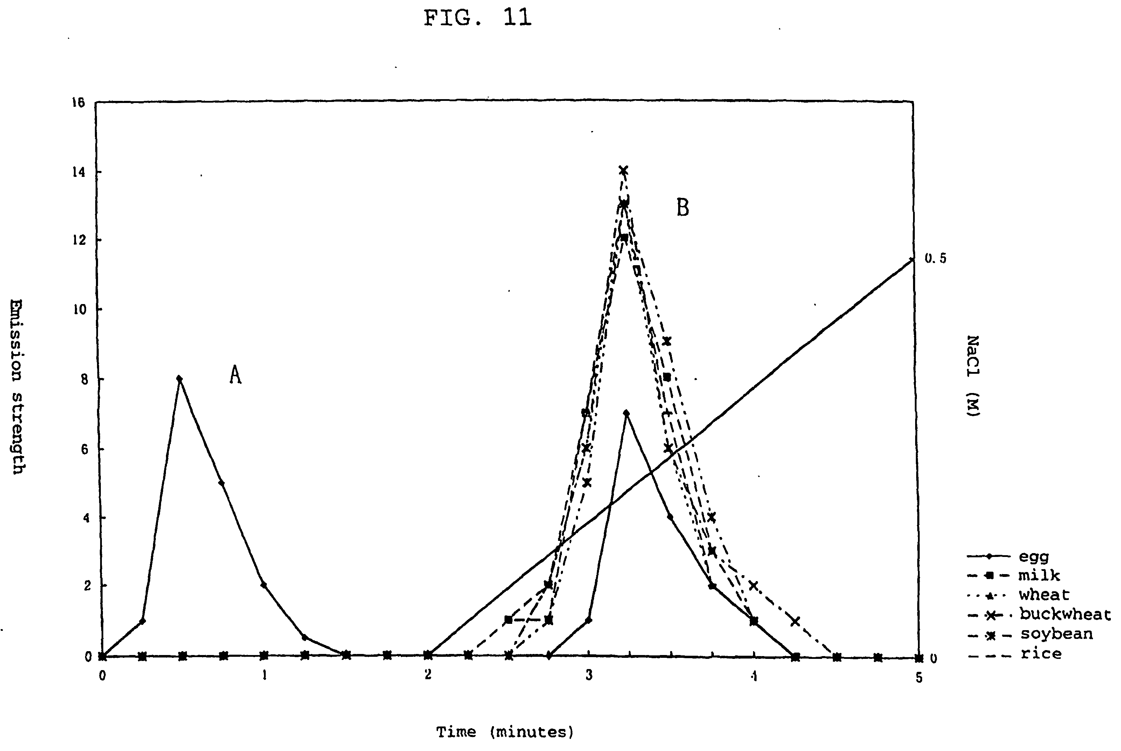

The results are shown in Fig. 11. In Fig.11, ◆ shows the results where the sample examined was the diluted albumen liquid, ■ shows the results where the sample examined was the β-lactoglobulin solution (milk), ▲ shows the results where the sample examined was the wheat extract, × shows the results where the sample examined was the buckwheat extract, * shows the results where the sample examined was the soybean extract, and - shows the results where the sample examined was the rice extract. When the anti-albumen allergen-IgG antibody was used, the albumen allergen labeled antibody complex was properly separated from the unreacted labeled antibody, and thus the allergen labeled antibody complex was able to be analyzed. Further, when the sample was the β-lactoglobulin solution (milk), the wheat extract, the buckwheat extract, the soybean extract, or the rice extract, only the unreacted labeled anti-albumen allergen-IgG antibody was eluted.

### (3) Measurement of allergen by anti-rice allergen-IgG antibody

The procedure of Example 6(1) was repeated except that the rice extract prepared in Example 6(1) was used as an antigen instead of β-lactoglobulin, to thereby obtain an anti-rice allergen-IgG antibody fraction. Then an alkaline phosphatase labeled-anti-rice allergen-IgG antibody was prepared, using the resulting IgG antibody fraction. An antigen-antibody reaction was carried out with the diluted albumen liquid, the β-lactoglobulin solution, the wheat extract, the buckwheat extract, the soybean extract or the rice extract prepared in Example 6(1), and the antigen-antibody was analyzed.

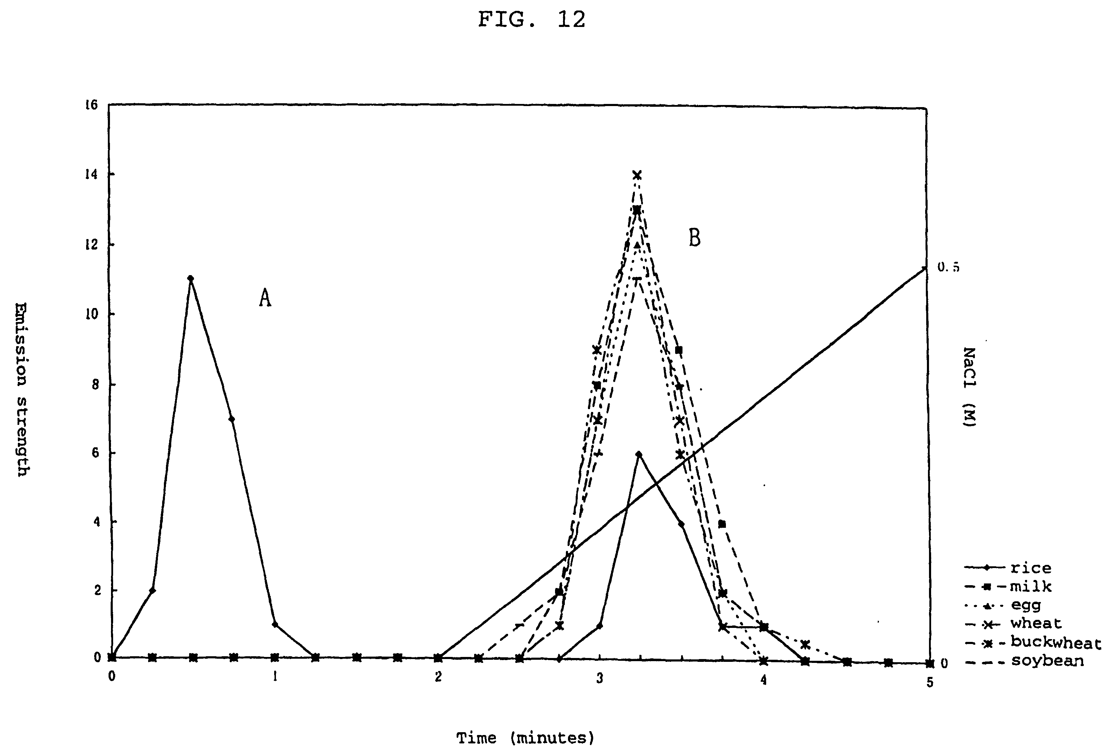

The results are shown in Fig. 12. In Fig. 12 ◆ shows the results where the sample examined was the rice extract, ■ shows the results where the sample examined was the β-lactoglobulin solution (milk), ▲ shows the results where the sample examined was the diluted albumen liquid, × shows the results where the sample examined was the wheat extract, * shows the results where the sample examined was the buckwheat extract, and - shows the results where the sample examined was the soybean extract. When the anti-rice allergen-IgG antibody was used, the rice allergen labeled antibody complex was properly separated from the unreacted labeled antibody, and thus the allergen labeled antibody complex was able to be analyzed. Further, when the sample was the β-lactoglobulin solution (milk), the diluted albumen liquid, the wheat extract, the buckwheat extract, or the soybean extract, only the unreacted labeled anti-rice allergen-IgG antibody was eluted.

### (4) Measurement of allergen by anti-wheat allergen-IgG antibody

The procedure of Example 6(1) was repeated except that the wheat extract prepared in Example 6(1) was used as an antigen instead of β-lactoglobulin, to thereby obtain an anti-wheat allergen-IgG antibody fraction. Then an alkaline phosphatase labeled-anti-wheat allergen-IgG antibody was prepared, using the resulting IgG antibody fraction. An antigen-antibody reaction was carried out with the diluted albumen liquid, the β-lactoglobulin solution, the wheat extract, the buckwheat extract, the soybean extract or the rice extract prepared in Example 6(1), and the antigen-antibody was analyzed.

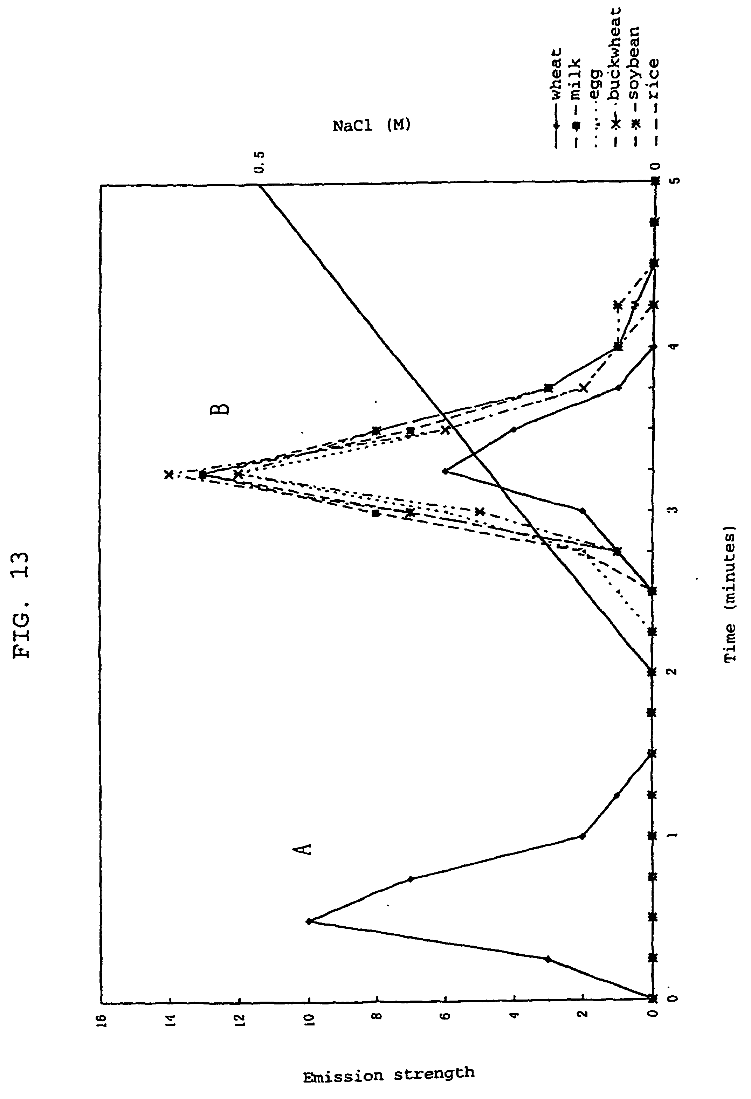

The results are shown in Fig. 13. In Fig.13, ◆ shows the results where the sample examined was the wheat extract, ■ shows the results where the sample examined was the β-lactoglobulin solution (milk), ▲ shows the results where the sample examined was the diluted albumen liquid, × shows the results where the sample examined was the buckwheat extract, * shows the results where the sample examined was the soybean extract, and - shows the results where the sample examined was the rice extract. When the anti-wheat allergen-IgG antibody was used, the wheat allergen labeled antibody complex was properly separated from the unreacted labeled antibody, and thus the allergen labeled antibody complex was able to be analyzed. Further, when the sample was the β-lactoglobulin solution (milk), the diluted albumen liquid, the buckwheat extract, the soybean extract, or the rice extract, only the unreacted labeled anti-wheat allergen-IgG antibody was eluted.

### (5) Measurement of allergen by anti-buckwheat allergen-IgG antibody

The procedure of Example 6(1) was repeated except that the buckwheat extract prepared in Example 6(1) was used as an antigen instead of β-lactoglobulin, to thereby obtain an anti-buckwheat allergen-IgG antibody fraction. Then, an alkaline phosphatase labeled-anti-buckwheat allergen-IgG antibody was prepared, using the resulting IgG antibody fraction. An antigen-antibody reaction was carried out with the diluted albumen liquid, the β-lactoglobulin solution, the wheat extract, the buckwheat extract, the soybean extract or the rice extract prepared in Example 6(1), and the antigen-antibody was analyzed.

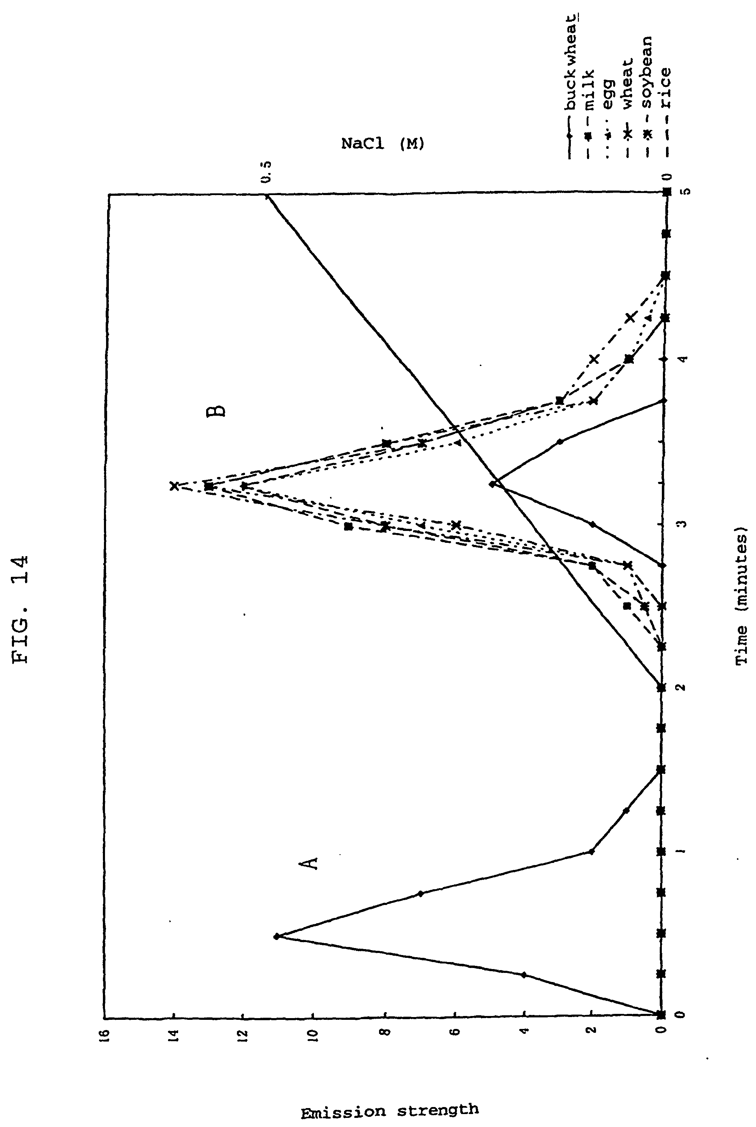

The results are shown in Fig. 14. In Fig.14, ◆ shows the results where the sample examined was the buckwheat extract, ■ shows the results where the sample examined was the β-lactoglobulin solution (milk), ▲ shows the results where the sample examined was the diluted albumen liquid, × shows the results where the sample examined was the wheat extract, * shows the results where the sample examined was the soybean extract, and - shows the results where the sample examined was the rice extract. When the anti-buckwheat allergen-IgG antibody was used, the buckwheat allergen labeled antibody complex was properly separated from the unreacted labeled antibody, and thus the allergen labeled antibody complex was able to be analyzed. Further, when the sample was the β-lactoglobulin solution (milk), the diluted albumen liquid, the wheat extract, the soybean extract, or the rice extract, only the unreacted labeled anti-buckwheat allergen-IgG antibody was eluted.

### (6) Measurement of allergen by anti-soybean allergen-IgG antibody

The procedure of Example 6(1) was repeated except that the soybean extract prepared in Example 6(1) was used as an antigen instead of β-lactoglobulin, to thereby obtain an anti-soybean allergen-IgG antibody fraction. Then an alkaline phosphatase labeled-anti-soybean allergen-IgG antibody was prepared, using the resulting IgG antibody fraction. An antigen-antibody reaction was carried out with the diluted albumen liquid, the β-lactoglobulin solution, the wheat extract, the buckwheat extract, the soybean extract or the rice extract prepared in Example 6(1), and the antigen-antibody was analyzed.

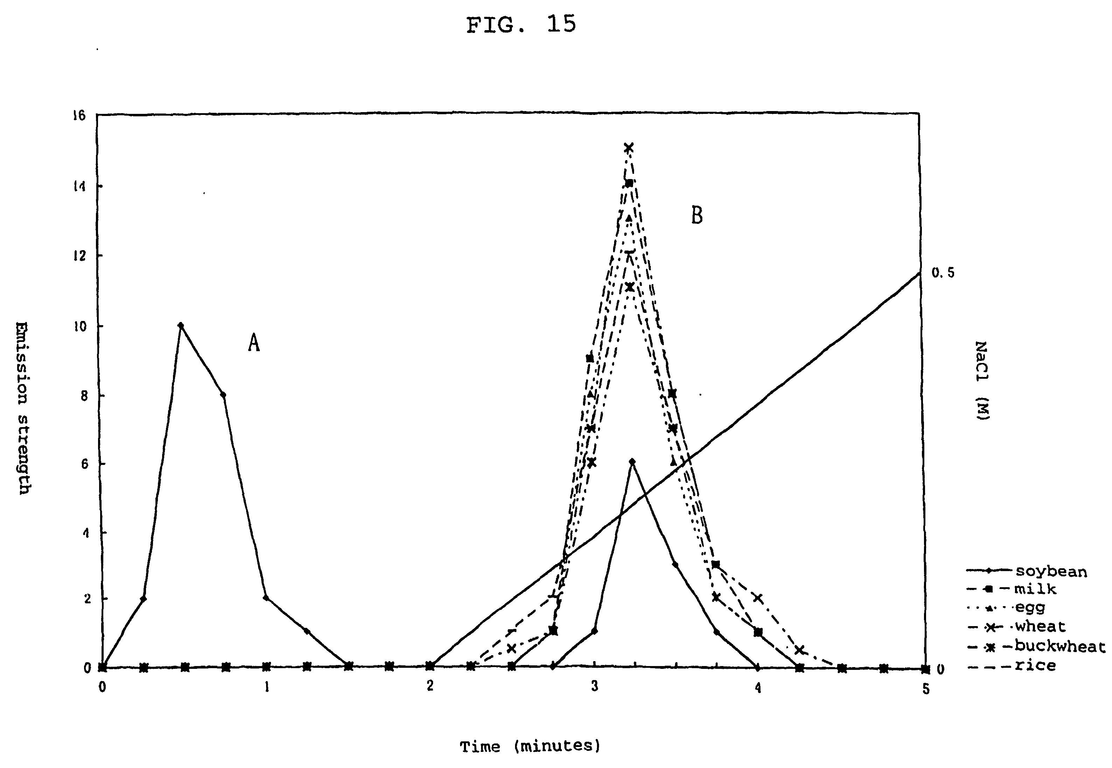

The results are shown in Fig. 15. In Fig. 15, ◆ shows the results where the sample examined was the soybean extract, ■ shows the results where the sample examined was the β-lactoglobulin solution (milk), ▲ shows the results where the sample examined was the diluted albumen liquid, × shows the results where the sample examined was the wheat extract, * shows the results where the sample examined was the buckwheat extract, and - shows the results where the sample examined was the rice extract. When the anti-soybean allergen-IgG antibody was used, the soybean allergen labeled antibody complex was properly separated from the unreacted labeled antibody, and thus the allergen labeled antibody complex was able to be analyzed. Further, when the sample was the β-lactoglobulin solution (milk), the diluted albumen liquid, the wheat extract, the buckwheat extract, or the rice extract, only the unreacted labeled anti-soybean allergen-IgG antibody was eluted.

### Example 7: Evaluation of quantifiability

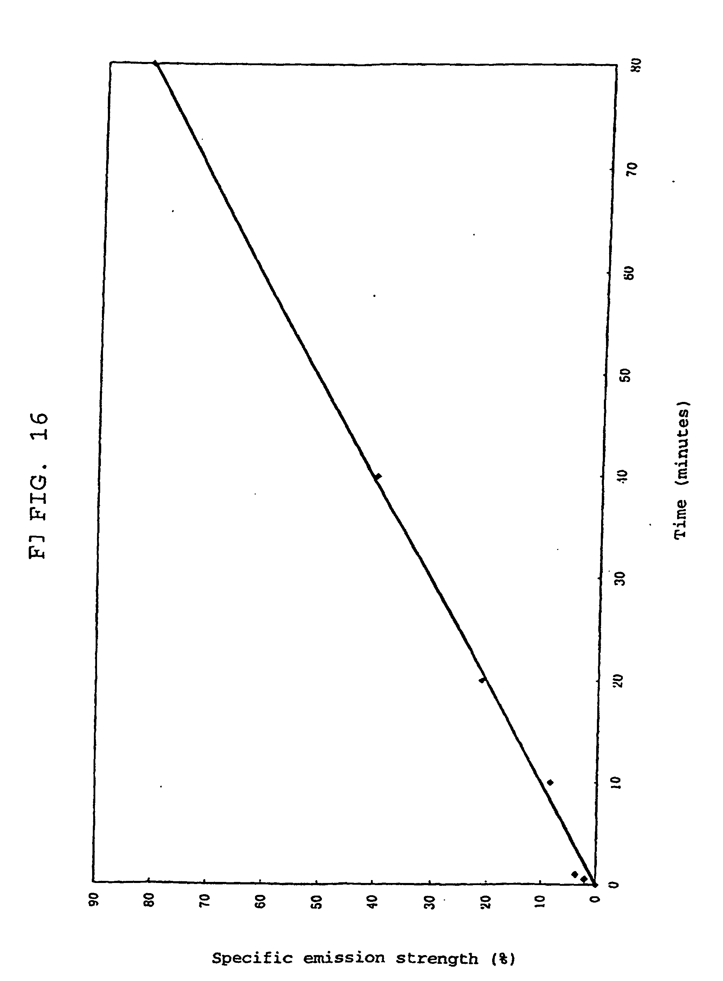

The quantifiability (capability of quantitative determination) was evaluated, by carrying out the procedures disclosed in Examples 4(3) and 4(4), using the solution containing the known amount of β-lactoglobulin and the alkaline phosphatase labeled anti-β-lactoglobulin allergen-IgG antibody of Example 6(1). The results are shown in Fig. 16. Fig. 16 shows a relative emission strength calculated to a 100% emission strength obtained by adding the whole sample into the column.

A linear relation was observed in the range where the concentration of the β-lactoglobulin was 0 to 80 µg/ml. As above, it was confirmed that an allergen can be quantitatively determined in the method of the present invention.

### Example 8: Assay of food allergen with FITC labeled IgE antibody

The procedure in Example 4(2) was repeated, except that a serum from a patient suffering from a milk, albumen, rice, wheat, buckwheat or soybean allergy was used, to thereby obtain an IgE antibody, respectively. Further, the procedure in Example 1(1) was repeated except that the resulting IgE antibody was used, to thereby obtain an FITC labeled IgE antibody.

As a sample, milk, or the diluted albumen liquid, the wheat extract, the buckwheat extract, soybean extract or rice extract prepared in Example 6(1) was used. The FITC labeled IgE antibody (1.5 ng) from the patient suffering from an allergy was reacted with the sample (2 mg of allergen protein) at 37°C for 20 minutes to perform an antigen-antibody reaction. According to the procedure in Example 1(3), the fluorescent strength from the allergen labeled antibody complex was measured for each reaction liquor.

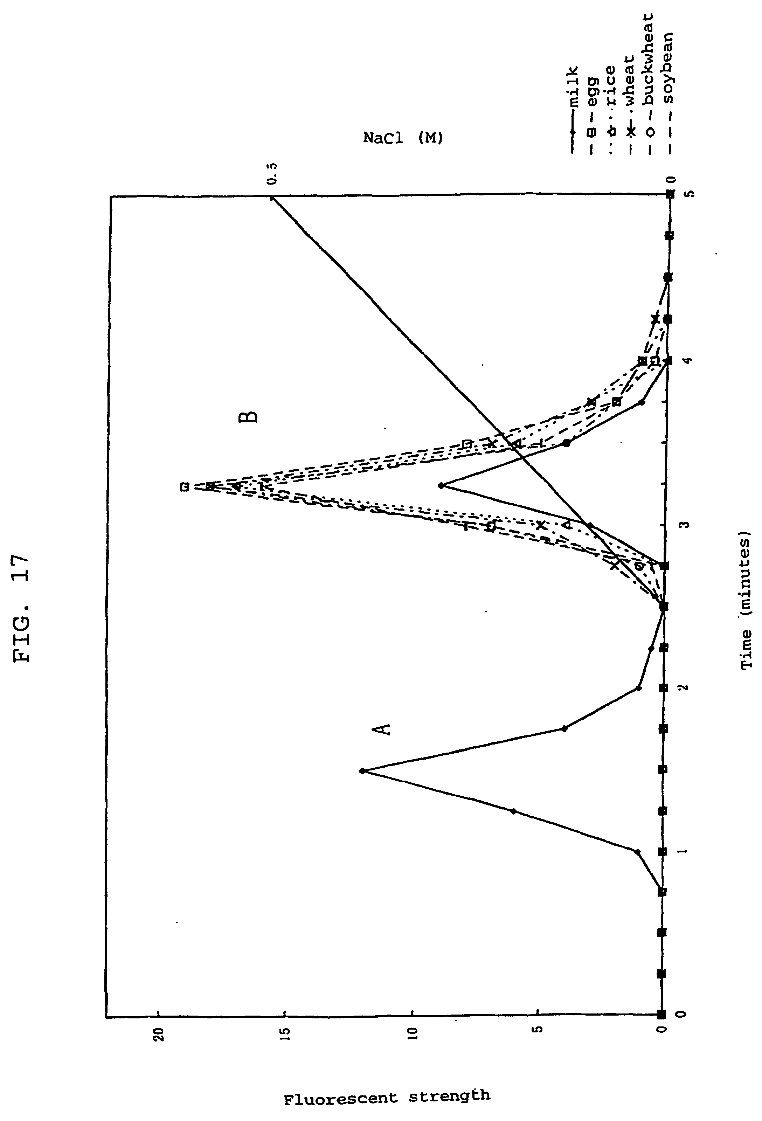

Fig. 17 shows the results when the FITC labeled-IgE antibody prepared from the IgE antibody derived from the patient suffering from a milk allergy was used. The symbols in Fig. 17 have the same meanings as explained in Fig. 10. As shown in Fig. 17, when the FITC labeled-IgE antibody prepared from the IgE antibody derived from the patient suffering from a milk allergy was used, the fluorescence from the allergen labeled antibody complex was detected only for milk. Thus, it was determined that the patient exhibited an allergy to milk.

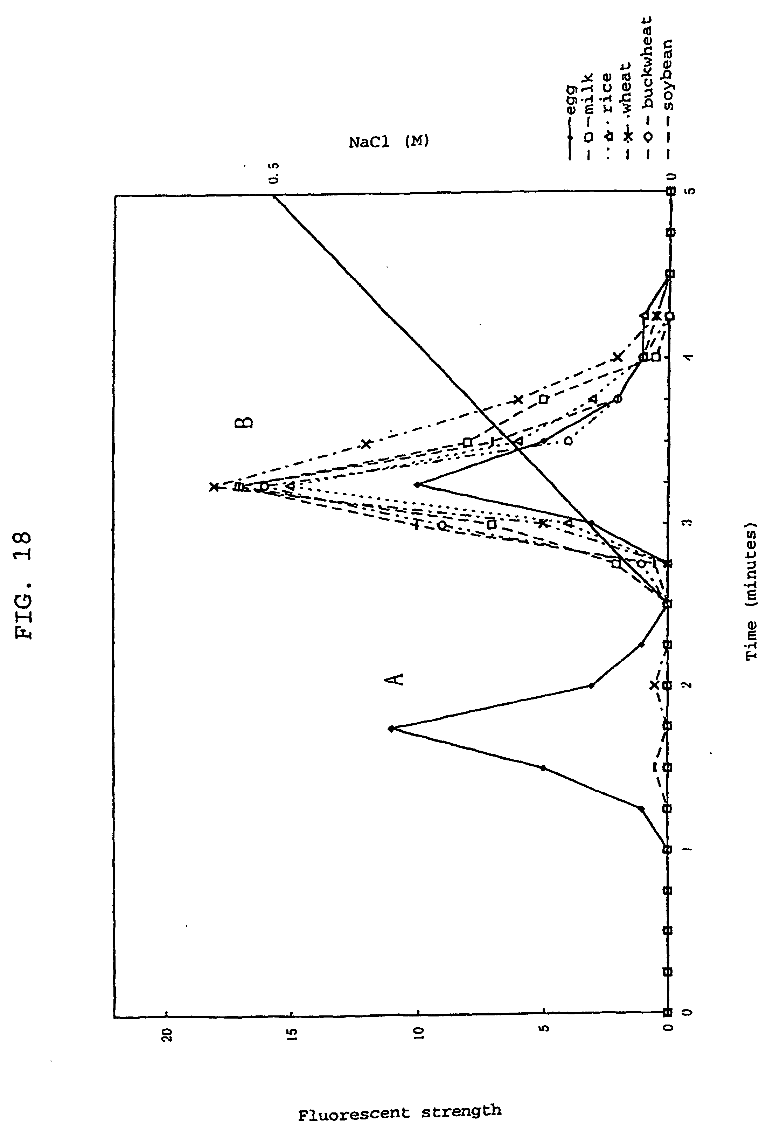

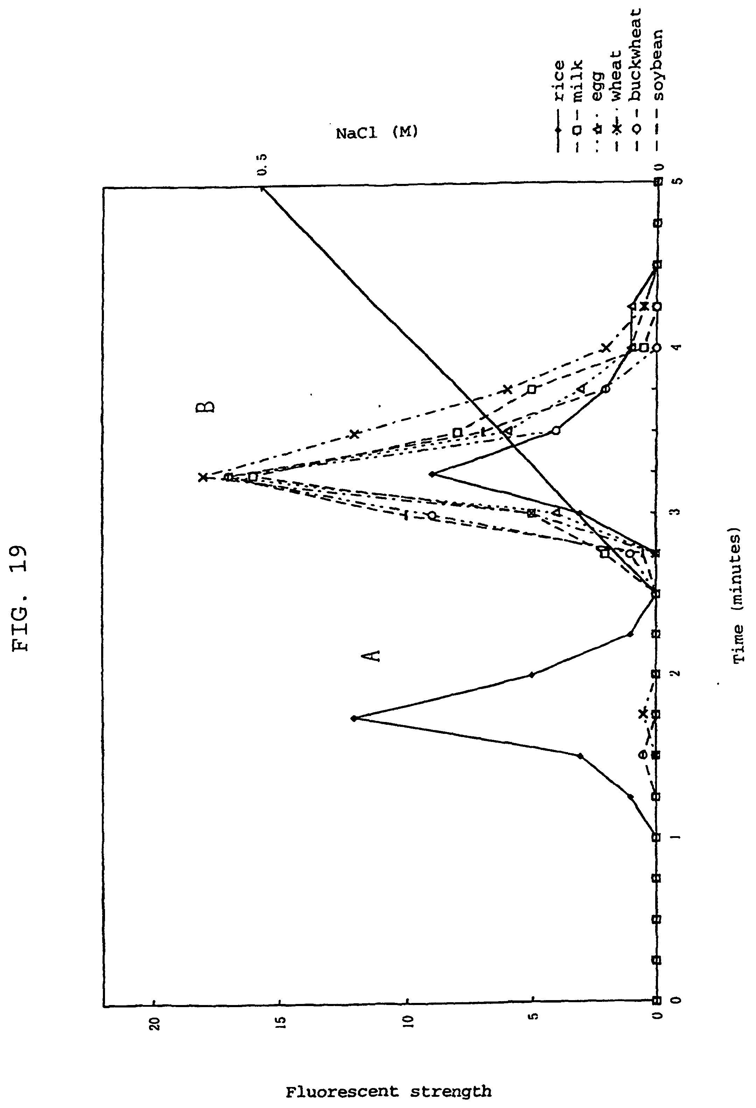

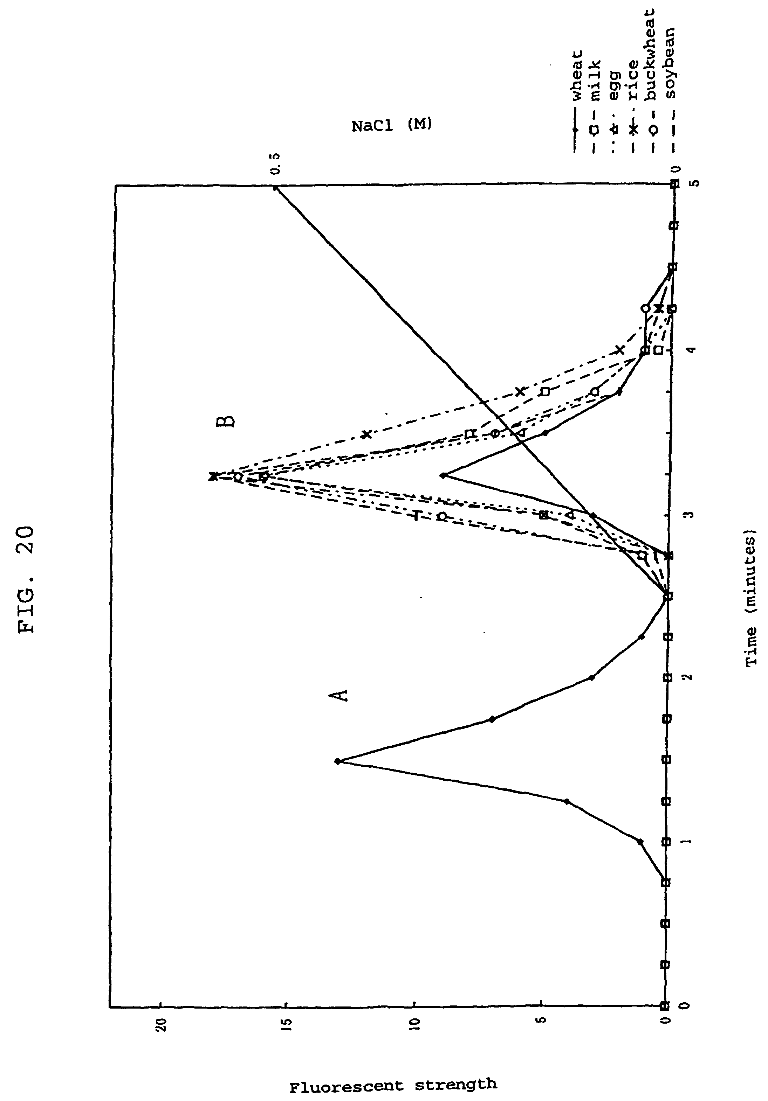

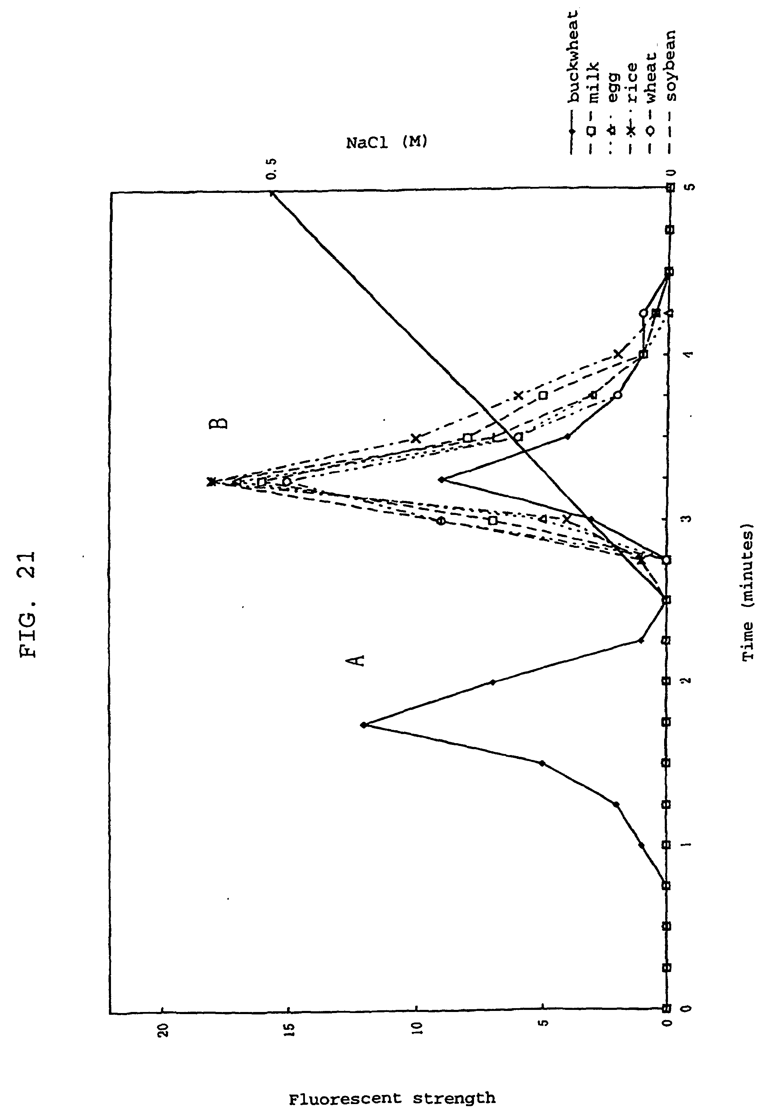

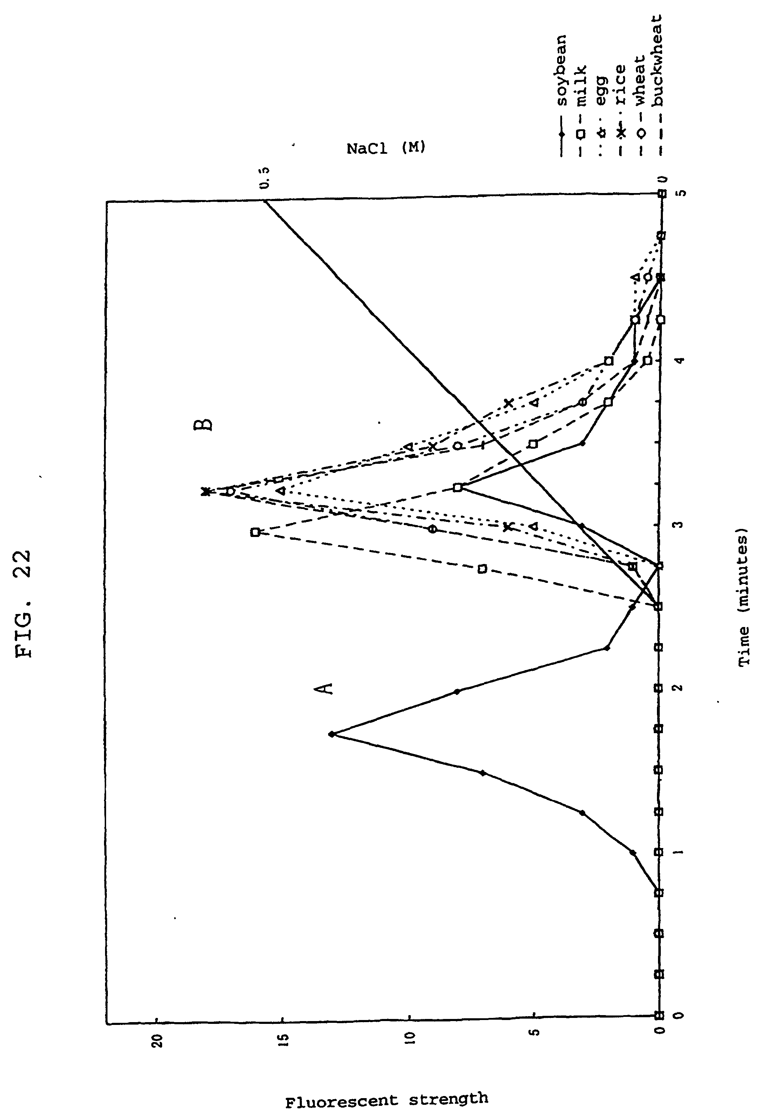

The results when the FITC labeled-IgE antibody prepared from the IgE antibody derived from each of the patients suffering from an egg, rice, wheat, buckwheat or soybean allergy was used are shown in Fig. 18 (when the IgE antibody from the patient suffering from an egg allergy was used), Fig. 19 (when the IgE antibody from the patient suffering from a rice allergy was used), Fig. 20 (when the IgE antibody from the patient suffering from a wheat allergy was used), Fig. 21 (when the IgE antibody from the patient suffering from a buckwheat allergy was used), and Fig. 22 (when the IgE antibody from the patient suffering from a soybean allergy was used).

As shown in Figs. 18 to 22, when the FITC labeled-IgE antibody prepared from the IgE antibody derived from the patient suffering from an egg, rice, wheat, buckwheat or soybean allergy was used, the fluorescence from the allergen labeled antibody complex was detected only for egg, rice, wheat, buckwheat or soybean, and thus, an allergy from which the patient suffered was identified.

### Example 9: Assay of allergen by successive addition to column

100 µl of a 1.8 ng/µl FITC labeled anti-DNP-IgE antibody prepared in Example 1(1) and 100 µl of a dinitrophenylated bovine serum albumin (DNP-BSA; 0.6 mg/ml, 1.0 mg/ml, 1.3 mg/ml or 2.0 mg/ml) were mixed, and the mixture was incubated at 37 °C for 20 minutes. As a control test (blank), the same procedure was repeated except that water was used instead of the DNP-BSA aqueous solution. Thereafter, 100 µl of the reaction liquor was applied into a cation exchange column which had been equilibrated with a 0.5 mM malonate buffer (pH 5.0) and used in Example 1(3), at an interval of 20 minutes. The column was not washed with saline between the application of the samples. Samples of DNP-BSA were added 4 times for each concentration, respectively. As a mobile phase, a 50 mM malonate buffer (pH 5.0) was used at a flow rate of 1 ml/min. The eluted liquid was divided into fractions of 0.25 ml. A fluorescent-spectrophotometer (F-2000; manufactured by Hitachi Ltd.) was used to conduct a detection by measuring a fluorescent strength of the eluted liquid.

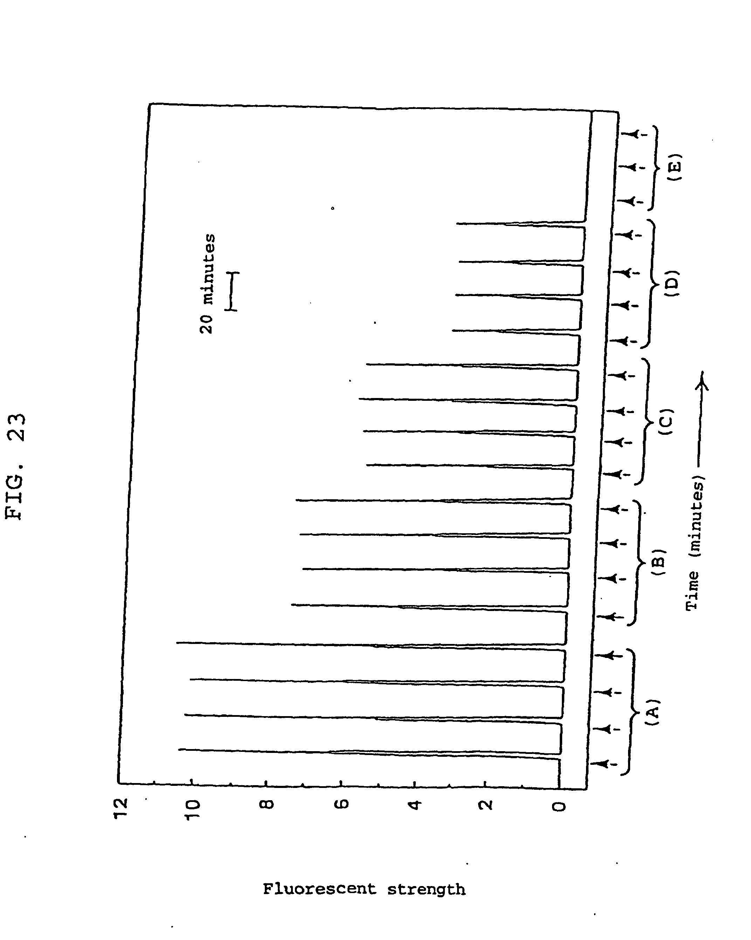

The resulting fluorescent strength of the eluted antigen-antibody complex was as shown in Fig. 23. In Fig. 23, "↑" shows the time when each reaction liquor was added, "A" means the addition of the reaction liquor mixed with 2.0 mg/ml DNP-BSA, "B" means the addition of the reaction liquor mixed with 1.3 mg/ml DNP-BSA, "C" means the addition of the reaction liquor mixed with 1.0 mg/ml DNP-BSA, "D" means the addition of the reaction liquor mixed with 0.6 mg/ml DNP-BSA, and "E" means the addition of the reaction liquor mixed with water. Stable values were observed in each concentration of DNP-BSA. It was confirmed that, even if the column is not washed with saline in a successive application of the samples, a quantitative determination can be achieved within the range that the ion exchange resin used was able to adsorb the unreacted FITC labeled IgE antibodies.

### Example 10: Assay of unreacted antibodies by anion exchange column

The anti-DNP-IgE antibody was labeled with FITC by the procedure in Example 1(1). Then, 100 µl of the FITC labeled anti-DNP-IgE antibody (1.8 ng/µl) and 100 µl of DNP-BSA (model allergen; 2 mg/ml) were reacted at room temperature for 20 minutes.

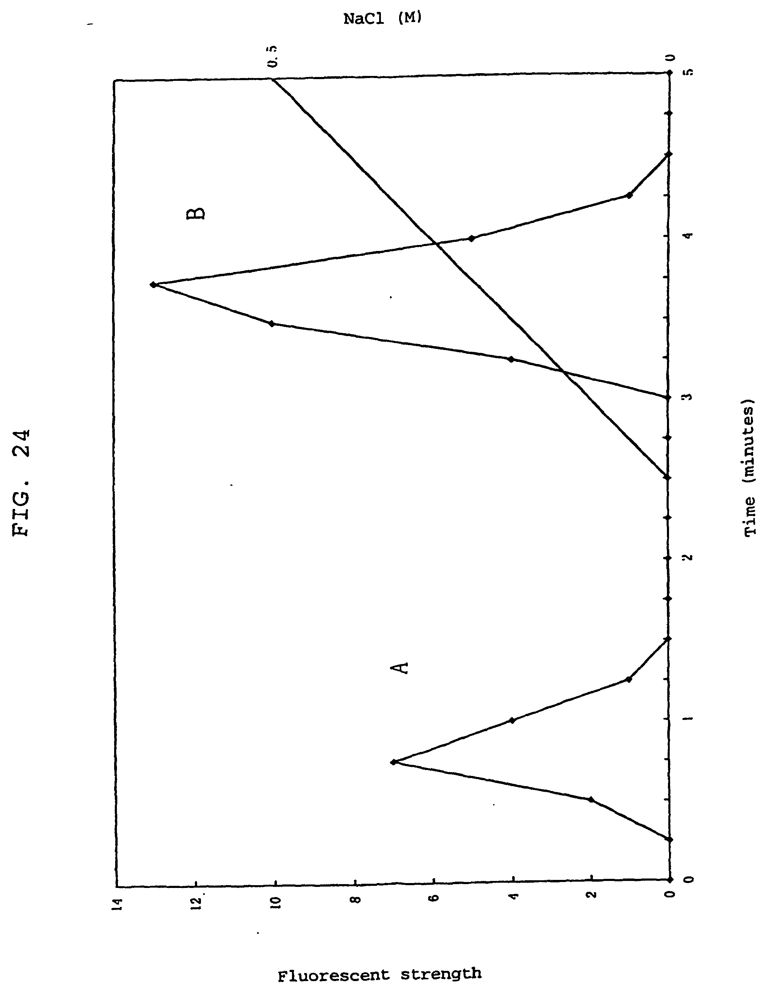

The reaction liquor was added to an anion exchange column (HILOAD Q Sepharose Fast Flow; diameter = 16 x 25 mm; manufactured by Pharmacia) which had been equilibrated with a 50 mM glycine-sodium chloride-sodium hydroxide buffer (pH 9). As a mobile phase, the buffer (pH 9.0) used for the equilibration was used at a flow rate of 1 ml/min for 2.5 minutes. Thereafter, an NaCl aqueous solution with a linear concentration gradient of 0 to 0.5 M was passed through. The eluted liquor was divided into fractions of 0.25 ml, and fluorescent-spectrophotometer (F-2000; manufactured by Hitachi Ltd.) was used to measure a fluorescent strength of the eluted liquid. The results are shown in Fig. 24. As shown in Fig. 24, only the allergen labeled antibody complex (peak A) was eluted in the 50 mM glycine-sodium chloride-sodium hydroxide buffer eluted fraction, and the unreacted FITC labeled IgE antibody (peak B) was eluted when the NaCl aqueous solution with the linear concentration gradient of 0 to 0.15 M was passed. Namely, when the anion exchanger was used instead of the cation exchanger, the product of the antigen-antibody reaction and the unreacted labeled antigen were properly separated, and the antigen-antibody complex alone was able to be analyzed.

### INDUSTRIAL APPLICABILITY

According to the present invention, an allergen can be readily and quickly quantitatively determined, different from conventional methods.

As shown above, the present invention has been explained with reference to particular embodiments, but modifications and improvements obvious to those skilled in the art are included in the scope of the present invention.

## Claims

1. A method for assaying an allergen, **characterized by** comprising the steps of:
(1) bringing a sample possibly containing an allergen to be examined into contact with a labeled antibody specific to the allergen to be examined under a condition wherein an antigen-antibody reaction between the allergen to be examined and the labeled antibody can be carried out;
(2) removing the labeled antibody not bound to the allergen to be examined, from a reaction mixture by means of an ion exchange chromatography, to obtain an eluted complex of the allergen to be examined and the labeled antibody from the ion exchange chromatography; said ion exchange chromatography being carried out under a condition that the complex of the allergen to be examined and the labeled antibody is not adsorbed on an ion exchanger in a range where the unreacted labeled antibody is adsorbed on the ion exchanger; and
(3) measuring a signal from the eluted complex of the allergen to be examined and the labeled antibody.

2. The method according to claim 1, wherein the label is a fluorescent compound, enzyme, or radioactive material.

3. The method according to claim 2, wherein fluorescein isothiocyanate is used as the fluorescent compound.

4. The method according to claim 2, wherein alkaline phosphatase, β-D-galactosidase, horseradish peroxidase, or catechol-o-methyltransferase is used as the enzyme.

5. The method according to claim 1, wherein a cation exchange chromatography is used as the ion exchange chromatography.

6. The method according to claim 5, wherein the unreacted labeled antibody is adsorbed to a cation exchanger and the complex of the allergen to be examined and the labeled antibody is eluted with an acidic buffer.

7. The method according to claim 6, wherein a pH of the acidic buffer is 4.5 to 6.

8. The method according to claim 1, wherein an anion exchange chromatography is used as the ion exchange chromatography.

9. The method according to any one of claims 1 to 8, wherein the antibody is IgE or IgG.

10. The method according to any one of claims 1 to 8, wherein the steps (1) to (3) are continuously repeated.

## Patentansprüche

1. Verfahren zur Untersuchung auf ein Allergen, **dadurch gekennzeichnet, dass** es die Stufen:
1) In-Berührung-Bringen einer Probe, die möglicherweise ein zu untersuchendes Allergen enthält, mit einem für das zu untersuchende Allergen spezifischen markierten Antikörper unter Bedingungen, bei welchen eine Antigen-Antikörper-Reaktion des zu untersuchenden Allergens mit dem markierten Antikörper stattfinden kann,
2) Entfernen des nicht an das zu untersuchende Allergen gebundenen, markierten Antikörpers aus einem Reaktionsgemisch durch Ionenaustauschchromatographie, um dadurch einen eluierten Komplex aus dem zu untersuchenden Allergen und dem markierten Antikörper zu erhalten, wobei die Ionenaustauschchromatographie unter solchen Bedingungen durchgeführt wird, dass der Komplex aus zü untersuchendem Allergen und markiertem Antikörper an einen Ionenaustauscher nicht innerhalb eines Bereichs adsorbiert wird, in welchem der nicht umgesetzte markierte Antikörper an den Ionenaustauscher adsorbiert wird, und
3) Messen eines Signals des eluierten Komplexes aus dem zu untersuchenden Allergen und dem markierten Antikörper umfasst.

2. Verfahren nach Anspruch 1, wobei die Markierung eine fluoreszierende Verbindung, ein Enzym oder ein radioaktives Material ist.

3. Verfahren nach Anspruch 2, wobei Fluoresceinisothiocyanat als fluoreszierende Verbindung verwendet wird.

4. Verfahren nach Anspruch 2, wobei alkalische Phosphatase, β-D-Galactosidase, Meerrettichperoxidase oder Catechol-o-methyltransferase als Enzym verwendet wird.

5. Verfahren nach Anspruch 1, wobei eine Kationenaustauschromatographie als Ionenaustauschromatographie angewendet wird.

6. Verfahren nach Anspruch 5, wobei der nicht umgesetzte markierte Antikörper an einen Kationenaustauscher adsorbiert und der Komplex aus dem zu untersuchenden Allergen und dem markierten Antikörper mit einem sauren Puffer eluiert wird.

7. Verfahren nach Anspruch 6, worin der pH-Wert des sauren Puffers 4,5 bis 6 beträgt.

8. Verfahren nach Anspruch 1, wobei eine Anionenaustauschromatographie als Ionenaustauschromatographie angewendet wird.

9. Verfahren nach einem der Ansprüche 1 bis 8, worin der Antikörper IgE oder IgG ist.

10. Verfahren nach einem der Ansprüche 1 bis 8, worin die Stufen 1) bis 3) kontinuierlich wiederholt werden.

## Revendications

1. Méthode d'analyse d'un allergène, **caractérisée en ce qu'**elle comprend les étapes consistant :
(1) à mettre un échantillon à étudier, pouvant contenir un allergène, en contact avec un anticorps marqué spécifique de l'allergène à étudier dans des conditions dans lesquelles une réaction antigène-anticorps entre l'allergène à étudier et l'anticorps marqué peut être conduite ;
(2) à éliminer l'anticorps marqué non lié à l'allergène à étudier, d'un mélange réactionnel par chromatographie d'échange d'ions pour obtenir un complexe élué de l'allergène à étudier et de l'anticorps marqué provenant de la chromatographie d'échange d'ions ; ladite chromatographie d'échange d'ions étant mise en oeuvre dans des conditions telles que le complexe de l'allergène à étudier et de l'anticorps marqué ne soit pas adsorbé sur un échangeur d'ions dans un intervalle où l'anticorps marqué n'ayant pas réagi est adsorbé sur l'échangeur d'ions ; et
(3) à mesurer un signal provenant du complexe élué de l'allergène à étudier et de l'anticorps marqué.

2. Méthode suivant la revendication 1, dans laquelle le marqueur est un composé fluorescent, une enzyme ou une matière radioactive.

3. Méthode suivant la revendication 2, dans laquelle de l'isothiocyanate de fluorescéine est utilisé comme composé fluorescent.

4. Méthode suivant la revendication 2, dans laquelle de la phosphatase alcaline, de la β-D-galactasidase, de la peroxydase de raifort ou de la catéchol-o-méthyltransférase est utilisée comme enzyme.

5. Méthode suivant la revendication 1, dans laquelle une chromatographie d'échange de cations est utilisée comme chromatographie d'échange d'ions.

6. Méthode suivant la revendication 5, dans laquelle l'anticorps marqué n'ayant pas réagi est adsorbé à un échangeur de cations et le complexe de l'allergène à étudier et de l'anticorps marqué est élué avec un tampon acide.

7. Méthode suivant la revendication 6, dans lequel un pH du tampon acide est compris dans la plage de 4,5 à 6.

8. Méthode suivant la revendication 1, dans laquelle une chromatographie d'échange d'anions est utilisée comme chromatographie d'échange d'ions.

9. Méthode suivant l'une quelconque des revendications 1 à 8, dans laquelle l'anticorps est une IgE ou une IgG.

10. Méthode suivant l'une quelconque des revendications 1 à 8, dans laquelle les étapes (1) à (3) sont répétées dé manière continue.
